# EUROPEAN PATENT APPLICATION

(11) **EP 4 292 619 A1**
(43) Date of publication of application: **20.12.2023**
(21) Application number: 22752753.8
(22) Date of filing: 08.02.2022
(51) Int. Cl.: A61L 27/24, A61L 27/16, A61L 27/36, A61L 27/38, A61L 27/44, A61L 27/50, A61L 27/52, A61L 27/54, A61L 27/58

(54) **TRANSPLANTATION DEVICE**

(30) Priority: 09.02.2021 JP 2021019175
(71) Applicant: Shin-Etsu Chemical Co., Ltd., Tokyo 100-0005 (JP); Japan Vam & Poval Co., Ltd., Sakai-shi, Osaka 592-8331 (JP); Tohoku University, Sendai-shi, Miyagi 980-8577 (JP)
(72) Inventor: KANESATO Shuhei, Tokyo 100-0005 (JP); KIMURA Yoshihiro, Sakai-shi, Osaka 592-8331 (JP); OHARUDA Akinobu, Sakai-shi, Osaka 592-8331 (JP); ISHIBA Keita, Sakai-shi, Osaka 592-8331 (JP); GOTO Masafumi, Sendai-shi, Miyagi 980-8577 (JP)
(74) Representative: Lederer & Keller Patentanwälte Partnerschaft mbB
(86) International application number: PCT/JP2022/004983
(87) International publication number: WO 2022/172930

(57) **Abstract**

Provided is a novel device or a novel implantation device. The implantation device is one of the following: (1) an implantation device comprising a material (A) having a density of 12 mg/cm³ or more and a dissolution rate of 80% or less as determined after immersion of the material (A) in physiological saline at 37°C for 24 hours; and (2) an implantation device comprising a material (A) having a dissolution rate of 80% or less as determined after immersion of the material (A) in physiological saline at 37°C for 24 hours and a ratio of X/Y of 0.24 or more wherein X is a density (mg/cm³) of the material (A) and Y is a degree of swelling of the material (A) expressed in terms of fold increase as determined after immersion of the material (A) in physiological saline at 37°C for 60 minutes.

## Description

### TECHNICAL FIELD

The present invention relates to a novel implantation device and others.

### BACKGROUND ART

Cell- or tissue-containing devices (collectively may be called "cell-containing devices") have recently been studied for implantation therapy. Cell-containing devices are, for example, capable of holding living cells or tissue to supply hormones, proteins or other physiologically active substances involved in metabolic functions to a patient or to detoxify harmful substances in a patient body, thereby serving as an alternative organ in a human or animal patient suffering from a disease to prevent and/or treat the disease.

These devices may be composed of cells or tissue and serve as a cell- or tissue-containing device, and may further contain an immunoisolation layer or an immunoisolation function containing or embedding cells or tissue therein and serve as a device capable of transplanting cells or tissue embedded in an immunoisolation layer.

The devices having such an immunoisolation layer are advantageous in various aspects. For example, the immunoisolation layer is capable of protecting living cells or tissue from the immune defense system in the body, and hence the devices do not require administration of immunosuppressive drugs, in contrast to direct transplantation of cells or tissue, for example, living donor organ transplantation. The devices can therefore avoid adverse side effects associated with immunosuppressive drugs. The devices can also be implanted in a patient body in a less invasive manner. Lastly, the devices can solve the problem of organ donor shortage.

Such devices having an immunoisolation function may be in various forms, such as those in the form of a microcapsule or a macrocapsule formulation that encapsulates living cells or tissue in a polymer (e.g., a cell formulation). These types of devices have a strongly crosslinked polymer structure that is capable of protecting the cells or tissue from the immune defense system in the body. The devices also utilize the molecular permeability of the polymer to supply hormones or other substances secreted from the cells or tissue to the body of the recipient.

Several such devices are proposed in literature. For example, Patent literature 1 discloses a cell- or tissue-embedding device having an aqueous gel serving as an immunoisolation layer, the aqueous gel containing a modified polyvinyl alcohol resin having an active carbonyl group (A) and a crosslinking agent (B). Patent literature 2 discloses a cell- or tissue-embedding device having an aqueous gel serving as an immunoisolation layer, the aqueous gel containing a polyvinyl alcohol resin having a triad syndiotacticity of 32 to 40% (A).

However, living cells or tissue contained in these devices may not be supplied with sufficient oxygen or nutrients. The shortage of supply of oxygen or nutrients may be more noticeable for cell-containing devices containing naked living cells or tissue embedded in an immunoisolation layer. Live cells or tissue not supplied with sufficient oxygen or nutrients may cause central necrosis, and implantation of the cell-containing devices may become less effective, or the functions of the cell-containing devices may be severely deteriorated.

Such a problem often occurs when the devices are implanted in subcutaneous regions where blood vessels are not abundant. More severe shortage of oxygen or nutrients in subcutaneous regions will occur in large animals having thicker subcutaneous tissue.

To solve the problem of shortage of oxygen and nutrients, use of a cell growth factor has been proposed to induce angiogenesis in the transplantation site of live cells. For example, Patent literature 3 discloses use of a gelatin hydrogel containing a basic fibroblast growth factor.

### CITATION LIST

### PATENT LITERATURE

Patent literature 1: WO 2018/155621
Patent literature 2: WO 2018/155622
Patent literature 3: JP 2002-145797 A

### SUMMARY OF INVENTION

### TECHNICAL PROBLEM

An object of the present invention is to provide a novel device and others.

According to the studies conducted by the inventors, induction of angiogenesis only at the implantation site of a cell-containing device results in a small contact area between the cell-containing device and newly formed blood vessels, and oxygen or nutrients may not be efficiently supplied to living cells, living tissue, etc.

When angiogenesis is induced using cell growth factors, the blood and exudates from bleeding or inflammation may be accumulated at the angiogenesis site. Such blood and exudates form a liquid membrane between the newly formed blood vessels and cells or the cell-containing device, and the liquid membrane may prevent the diffusion of oxygen and nutrients from the newly formed blood vessels to the cell-containing device and may significantly inhibit the exhibition of the functions of the cell-containing device.

Accordingly, the inventors performed extensive studies focusing on a concept that is completely different from induction of angiogenesis by exogenous administration of growth factors as described in Patent literature 3, and found that implantation or placement of a particular device, material or member comprising a material having specific physical property values is capable of activating the implantation site. In particular, the inventors found that, for example, a device comprising a material having specific physical property values can be implanted or placed into an implantation site prior to implantation of a cell-containing device into the implantation site, thereby reducing or preventing the problems as described above and allowing the cell-containing device to efficiently exhibit its functions. The inventors conducted further studies and completed the invention.

Thus, the present invention relates to the following and others.
[1] An implantation device (or an implantation material or an implantation member) comprising a material (A) having a density of 12 mg/cm³ or more and a dissolution rate of 80% by mass or less as determined after immersion of the material (A) in physiological saline at 37°C for 24 hours.
[2] An implantation device comprising a material (A) having a dissolution rate of 80% or less as determined after immersion of the material (A) in physiological saline at 37°C for 24 hours and a ratio of X/Y of 0.22 or more wherein X is a density (mg/cm³) of the material (A) and Y is a degree of swelling of the material (A) expressed in terms of fold increase (i.e., fold increase by mass) calculated on mass basis after immersion of the material (A) in physiological saline at 37°C for 60 minutes.
[3] An implantation device comprising a material (A) having a density of 12 mg/cm³ or more, a dissolution rate of 80% by mass or less as determined after immersion of the material (A) in physiological saline at 37°C for 24 hours and a ratio of X/Y of 0.22 or more wherein X is a density (mg/cm³) of the material (A) and Y is a degree of swelling of the material (A) expressed in terms of fold increase calculated on mass basis after immersion of the material (A) in physiological saline at 37°C for 60 minutes.
[4] The device according to any one of the above [1] to [3], wherein the material (A) has a dissolution rate of 60% by mass or less as determined after immersion of the material (A) in physiological saline at 37°C for 24 hours.
[5] The device according to any one of the above [1] to [4], wherein the material (A) has a degree of swelling of 70-fold (i.e., 70-fold by mass) or less calculated on mass basis after immersion of the material (A) in physiological saline at 37°C for 60 minutes.
[6] The device according to any one of the above [1] to [5], wherein the material (A) has a ratio of X/Y of 0.23 or more wherein X is a density (mg/cm³) of the material (A) and Y is a degree of swelling of the material (A) expressed in terms of fold increase calculated on mass basis after immersion of the material (A) in physiological saline at 37°C for 60 minutes.
[7] The device according to any one of the above [1] to [6], wherein the material (A) has a density of 14 mg/cm³ or more, a dissolution rate of 3 to 50% by mass as determined after immersion of the material (A) in physiological saline at 37°C for 24 hours and a ratio of X/Y of 0.23 or more wherein X is a density (mg/cm³) of the material (A) and Y is a degree of swelling of the material (A) expressed in terms of fold increase (i.e., fold increase by mass) calculated on mass basis after immersion of the material (A) in physiological saline at 37°C for 60 minutes.
[8] The device according to any one of the above [1] to [7], wherein the material (A) contains a bioabsorbable material (A1).
[9] The device according to any one of the above [1] to [8], wherein the material (A) contains a gelatin.
[10] The device according to any one of the above [1] to [9], wherein the material (A) forms at least part of a surface of the device or is exposed on a surface of the device.
[11] The device according to any one of the above [1] to [10], wherein the device comprises a material (A) and a non-bioabsorbable material or member (B).
[12] The device according to any one of the above [1] to [11], wherein the device comprises a material (A) and a non-bioabsorbable material or member (B), in particular, wherein the material (A) contains a bioabsorbable material (A1), and wherein the material (A) is integrated with the non-bioabsorbable material or member (B).
[13] The device according to any one of the above [1] to [12], wherein the device comprises a material (A) and a non-bioabsorbable material or member (B), in particular, wherein the material (A) contains a bioabsorbable material (A1), and wherein the non-bioabsorbable material or member (B) is contained in an amount of 1 part by volume or more relative to 100 parts by volume of the material (A).
[14] The device according to any one of the above [1] to [13], wherein the device comprises a material (A) and a non-bioabsorbable material or member (B), in particular, wherein the material (A) contains a bioabsorbable material (A1), wherein the material (A) is integrated with the non-bioabsorbable material or member (B), wherein the non-bioabsorbable material or member (B) is contained in an amount of 10 parts by volume or more relative to 100 parts by volume of the material (A), and wherein the material (A) forms at least part of a surface of the device or is exposed on a surface of the device.
[15] The device according to any one of the above [11] to [14], wherein the non-bioabsorbable material or member (B) has an adhesion preventive ability.
[16] The device according to any one of the above [1] to [15], wherein the device is free of growth factors or is substantially free of growth factors.
[17] The device according to any one of the above [1] to [16], wherein the device is for formation of an encapsulating membrane.
[18] The device according to any one of the above [1] to [17], wherein the device is for implantation (or implantation or placement for a predetermined period of time) into an implantation site of a cell- or tissue-containing device, optionally prior to implantation of the cell- or tissue-containing device.
[19] The device according to any one of the above [1] to [18], wherein the device is for use in combination with a cell- or tissue-containing device.
[20] The device according to the above [18] or [19], wherein the cell- or tissue-containing device has an immunoisolation layer containing a polyvinyl alcohol resin (A).
[21] The device according to any one of the above [18] to [20], wherein the cell- or tissue-containing device comprises an immunoisolation layer containing at least one or more polyvinyl alcohol resins (A) selected from a modified polyvinyl alcohol resin having an active carbonyl group (A1), a polyvinyl alcohol resin having a triad syndiotacticity of 32 to 40% (A2), and a polyvinyl alcohol resin having a degree of saponification of 97 mol% or more (A3).
[22] The device according to any one of the above [18] to [22], wherein the cell or tissue contains at least one selected from a pancreatic islet or islet cells, hepatocytes, stem cells that give rise to islet cells or hepatocytes, and progenitor cells that give rise to islet cells or hepatocytes.
[23] A method for implanting (or implanting or placing for a predetermined period of time) the device according to any one of the above [1] to [22].
[24] A method for activating an implantation site, comprising implanting (or implanting or placing for a predetermined period of time) the device according to any one of the above [1] to [22] into the implantation site.
[25] The method according to the above [23] or [24], wherein a material containing a bioabsorbable material (A1) is used as a material (A), and wherein the bioabsorbable material (A1) or at least part of the bioabsorbable material (A1) is biologically absorbed, optionally at the implantation site.
[26] The method according to any one of the above [23] to [25], wherein a material containing a bioabsorbable material (A1) is used as a material (A) to allow formation of an encapsulating membrane, optionally at the implantation site.
[27] A method comprising implanting the device according to any one of the above [1] to [22], wherein the device comprises a non-bioabsorbable member as a material (A) or comprises a non-bioabsorbable material (B), wherein at least the non-bioabsorbable material is retrieved after implantation of the device (or after implantation or placement of the device for a predetermined period of time), and optionally wherein an implantation spot is created.
[28] The method according to the above [27], wherein the device is retrieved without causing bleeding, inflammation and/or rupture of a blood vessel, and optionally wherein an implantation spot is created.
[29] An implantation method comprising implanting a cell- or tissue-containing device into a site in which the device according to any one of the above [1] to [22] has been implanted and optionally subsequently retrieved.
[30] A method for preventing and/or treating a disease or a symptom, comprising implanting a cell- or tissue-containing device into a site in which the device according to any one of the above [1] to [22] has been implanted and optionally subsequently retrieved.
[31] The method according to the above [29] or [30], further comprising implanting the device according to any one of the above [1] to [22], optionally retrieving at least a non-bioabsorbable material or member, and optionally creating an implantation spot.
[32] The device or method according to any one of the above [1] to [31], wherein the implantation site is a subcutaneous site or subcutaneous tissue.

### ADVANTAGEOUS EFFECTS OF INVENTION

The present invention provides a novel device.

The device of the invention is capable of activating an implantation site or a placement site.

For example, the device of the invention can be implanted or placed into an implantation site prior to implantation of a cell-containing device into the implantation site, thereby reducing or preventing the problems as described above and allowing the cell-containing device to efficiently exhibit its functions.

When a material containing a bioabsorbable material, such as a gelatin or a collagen, is used as a material (A) to produce the device of the invention, an encapsulating membrane can be formed at the implantation site or the placement site or the neighboring site.

The reason why the device of the invention is capable of efficiently activating the implantation site remains unclear. However, the specific physical properties, e.g., the density and/or the dissolution rate, of a material contained in the device of the invention are adjusted to a predetermined range or value, and probably due to this, the device of the invention is capable of activating the implantation site through, e.g., the formation of a strong encapsulating membrane with an adequate thickness at the implantation site and/or other functions. The device of the invention is also capable of adjusting the implantation environment so that a subsequently implanted cell-containing device can easily perform its functions.

When a material containing a bioabsorbable material, such as a gelatin or a collagen, is used as a material (A) to produce the device of the invention, the material (A) is absorbed in the body and used to form part of an encapsulating membrane. Probably, in this manner, an encapsulating membrane with an adequate thickness is easily formed. The material (A) also tends to attract effector cells that secrete growth factors, and the attracted effector cells tend to accumulate in the material (A), which contributes to the effects of increasing the amount of endogenous extracellular matrix (ECM) and the levels of endogenous growth factors or of promoting the secretion of endogenous extracellular matrix (ECM) and endogenous growth factors.

The device of the invention may further comprise a non-bioabsorbable material. The non-bioabsorbable material may induce foreign body reactions, which may promote the formation of an encapsulating membrane and the secretion of growth factors.

Due to these functions, the device of the invention is suitable for use as an implantation device or a device for implantation of another device, and also as a device for formation of an encapsulating membrane, etc.

The device of the invention can be used for any applications. The device of the invention can be used for the desired application, e.g., for repair of an injury, e.g., for repair of tissue after surgery, for providing a scaffold of cells to be injected, or for other purposes depending on the type of device, the desired degree of activation or the desired manner (e.g., with or without the formation of an encapsulating membrane etc.).

The device of the invention is especially suitable as a device to be implanted (or to be implanted or to be placed for a predetermined period of time) into an implantation site of a cell-containing device, optionally prior to implantation of the cell-containing device.

The device of the invention used in this manner allows the cell-containing device to efficiently exhibit its functions or improves the functions of the cell-containing device. Such enhancement or improvement of the functions of the cell-containing device by the device of the invention is highly reproducible or highly accurate.

The device of the invention may be capable of activating the implantation site through, e.g., the formation of a strong encapsulating membrane with an adequate thickness at the implantation site, as described above, and may also be capable of improving the implantation environment to allow a subsequently implanted cell-containing device to easily perform its functions, although the reason is unknown.

When the device of the invention is capable of forming an encapsulating membrane, the encapsulating membrane facilitates close contact between the cell-containing device and the implantation site, and facilitates the efficient supply of oxygen and nutrients to living cells or tissue in the cell-containing device. In this manner, the device of the invention allows the cell-containing device to effectively exhibit its functions or improves the functions of the cell-containing device.

The encapsulating membrane may serve as a protection for the implantation site. For example, when a cell-containing device is implanted, the encapsulating membrane easily prevents the surrounding tissue from getting damaged by an instrument etc., and easily inhibits the secretion of cytokines etc. that may reduce the functions of the cell-containing device.

The encapsulating membrane also easily prevents an implanted cell-containing device from dislocating from the implantation site, thereby allowing the cell-containing device to efficiently perform its functions at the targeted site.

The device of the invention is also capable of activating the implantation site without using a growth factor or other factors. Such activation is completely different from so-called exogenous activation of the implantation site from outside the body by using a device containing a growth factor, as described in Patent literature 3.

In exogenous administration of growth factors, control of the release rate of growth factors is difficult, including sustained release and local administration, and the growth factors may diffuse from the implantation site to the systemic circulation. Accordingly, a large dose of growth factors may be required, or unexpected adverse side effects, e.g., induction of bleeding, swelling or tumorigenic transformation, may occur. Another disadvantage of exogenous administration is that targeted local increase or abundance of growth factors or other factors at the implantation site is difficult to achieve.

The device of the invention does not require such exogenous administration, and reduces or prevents these problems and efficiently activates the implantation site. When a cell-containing device is subsequently implanted, the device of the invention improves the functions of the cell-containing device.

The device of the invention is also useful in that the device is capable of being implanted into even a blood vessel-poor site, e.g., a subcutaneous site, in which a cell-containing device or other types of devices are assumed not to fully exhibit their functions, and the device of the invention is also capable of activating such a blood-vessel poor site.

For example, the device of the invention is capable of activating the implantation site of a cell-containing device as described above. Probably due to this activation, the device of the invention is capable of efficiently inhibiting or preventing central necrosis and greatly improving the functions of a cell-containing device, regardless of the abundance or scarcity of blood vessels or the presence or absence of angiogenesis at the implantation site.

The device of the invention does not require administration of exogenous growth factors or other factors as described above or subsequent angiogenesis by such exogenous growth factors or other factors, and is capable of efficiently preventing the accumulation of blood or exudates caused by bleeding or inflammation at the implantation site. The device of the invention therefore allows a cell-containing device or another device to fully exhibit its functions.

The device of the invention itself can induce angiogenesis at the implantation site, but even when angiogenesis is induced by the device, this angiogenesis is due to endogenous factors, and the degree of angiogenesis is smaller and less excessive than that induced by exogenous administration of growth factors. Therefore, even when bleeding or the like occurs, such bleeding or the like can usually be inhibited or prevented at a high level.

According to another aspect of the invention, the device of the invention comprises a non-bioabsorbable material. When such a device of the invention comprising a non-bioabsorbable material is implanted or placed in a body, at least the non-bioabsorbable material is usually not absorbed but remains in the body. Therefore, after retrieval of the device of the invention, a vacant space can be efficiently formed at the location where the non-bioabsorbable material has been placed, and serves as an implantation pocket.

Such an implantation pocket makes it easier to implant or locate a cell-containing device or the like in the body.

The device of the invention comprising such a non-bioabsorbable material can typically be efficiently retrieved. For example, typically, the device of the invention rarely develops adhesions to the implantation site or an encapsulating membrane formed or the surrounding sites. When the device of the invention is retrieved, the device rarely causes damage to these sites or the encapsulating membrane. In this manner, the device is capable of preventing or reducing bleeding, inflammation (induction of inflammation) or release of exudates from the implantation site etc. The device of the invention comprising such a non-bioabsorbable material is adapted to induce the activation of the implantation site, and is also adapted to facilitate the retrieval of the device, and is thus very useful.

### DESCRIPTION OF EMBODIMENTS

### Device

The device of the invention (may also be called the device 1, the material, the member, etc.) comprises at least a material (A).

### Material (A)

The material (A) or member (A) typically has specific physical property values.

For example, the density of the material (A) may be selected from the range of about 1 mg/cm³ or more, e.g., 5 mg/cm³ or more, or may be 10 mg/cm³ or more, e.g., 12 mg/cm³ or more, preferably about 14 mg/cm³ or more, e.g., 15 mg/cm³ or more, or may be 20 mg/cm³ or more, e.g., 25 mg/cm³ or more, 30 mg/cm³ or more, 35 mg/cm³ or more, 40 mg/cm³ or more, 45 mg/cm³ or more, 50 mg/cm³ or more, 55 mg/cm³ or more, etc.

The upper limit of the density of the material (A) may be, for example, but is not limited to, 5000 mg/cm³, 3000 mg/cm³, 2000 mg/cm³, 1500 mg/cm³, 1200 mg/cm³, 1000 mg/cm³, 800 mg/cm³, 700 mg/cm³, 600 mg/cm³, 500 mg/cm³, 400 mg/cm³, 350 mg/cm³, 300 mg/cm³, 250 mg/cm³, 200 mg/cm³, 180 mg/cm³, 150 mg/cm³, 120 mg/cm³, 100 mg/cm³, 90 mg/cm³, 80 mg/cm³, 70 mg/cm³, 60mg/cm³, etc.

When the material (A) is a bioabsorbable material, the density of the material (A) may not be excessively high, and, in particular, may be a rather smaller value, for example, 500 mg/cm³ or less, 300 mg/cm³ or less, 250 mg/cm³ or less, 200 mg/cm³ or less, 150 mg/cm³ or less, 100 mg/cm³ or less, 1 to 500 mg/cm³, 5 to 300 mg/cm³, 10 to 200 mg/cm³, 12 to 150 mg/cm³, 14 to 100 mg/cm³, etc.

The specific density range may appropriately be defined by the upper and lower limit values as described above, and may, for example, be 12 to 2000 mg/cm³, 15 to 1000 mg/cm³, etc. Hereinafter, the same applies to the reference to a range.

The density can be determined or measured from the volume and mass (weight) of the material (A).

In cases where the volume and mass of the material (A) cannot be directly measured, the volume and mass of the material (A) can be determined by an indirect measurement method. For example, the density of a material that has absorbed water or a water-absorbable material may be determined together with the measurement of the moisture content (moisture percentage) of the material by a conventional method. The measured moisture content is used to correct the density, or the moisture content is deducted to determine the density.

The material (A) having the density as described above, i.e., a relatively high density, facilitates the activation of the implantation site.

In particular, when an encapsulating membrane is formed, the material (A) with a relatively high density easily prevents the encapsulating membrane, e.g., fibroblasts etc. from entering the inside of the device, e.g., the pores or cavities of the device. The material (A) also facilitates efficient formation of an encapsulating membrane of the desired shape, an encapsulating membrane with a smooth surface, a strong encapsulating membrane, etc.

When the material (A) is made of a bioabsorbable material (A1) having the density as described above, the bioabsorbable material (A1) that has been absorbed in the body may form part of an encapsulating membrane, and such an encapsulating membrane may have an adequate thickness or a sufficient strength. The material (A) also tends to attract effector cells that secrete growth factors, and the attracted effector cells tend to accumulate in the material (A), which contributes to the effects of increasing the amount of endogenous extracellular matrix (ECM) and the levels of endogenous growth factors or of promoting the secretion of endogenous extracellular matrix (ECM) and endogenous growth factors.

When the material (A) is made of a non-bioabsorbable material having the density as described above, the device of the invention rarely develops adhesions to the surrounding tissue and is easily retrieved, and has other advantages.

The dissolution rate (the dissolution percentage or the residual percentage) of the material (A) is determined after immersion of the material (A) in physiological saline at 37°C for 24 hours and may be 90% by mass or less, e.g., 80% by mass or less, preferably 70% by mass or less, e.g., 60% by mass or less, more preferably 50% by mass or less, e.g., 45% by mass or less, or may be 40% by mass or less, e.g., 35% by mass or less, 32% by mass or less, 30% by mass or less, 28% by mass or less, 25% by mass or less, 22% by mass or less, 20% by mass or less, etc. The dissolution rate of the material (A) determined as above may also be 5% by mass or less, 3% by mass or less, 2% by mass or less, 1% by mass or less, 0% by mass, etc. depending on the type of material (A).

The lower limit of the dissolution rate may be selected depending on the type of material (A), etc., and may be, for example, 0% (no substantial dissolution), 0.5% by mass, 1% by mass, 2% by mass, 3% by mass, 5% by mass, 8% by mass, 10% by mass, 12% by mass, 14% by mass, 15% by mass, 16% by mass, 17% by mass, 18% by mass, etc.

Especially when the material (A) is a bioabsorbable material, the dissolution rate may not be too small but adequately large, and may be, for example, 14% by mass or more, 15% by mass or more, 16% by mass or more, 17% by mass or more, 18% by mass or more, 14 to 70% by mass, 15 to 50% by mass, 16 to 40% by mass, etc.

When the material (A) is made of a bioabsorbable material as described later, the lower limit of the dissolution rate is a finite value in most cases. When the material (A) is made of a non-bioabsorbable material, the dissolution rate is 0% by mass or a small percentage in most cases, and may be, e.g., 5% by mass or less, 3% by mass or less, 1% by mass or less, etc.

The material (A) having the dissolution rate as described above facilitates the activation of the implantation site.

In particular, when the material (A) is made of a bioabsorbable material (A1) having the dissolution rate as described above, the material (A) is slowly absorbed into the body or degraded and incorporated into the body and tends to form a thick encapsulating membrane with an adequate thickness. The material (A) also tends to hold effector cells for a longer period of time, which contributes to the effects of increasing the amount of endogenous extracellular matrix (ECM) and the levels of endogenous growth factors or of promoting the secretion of endogenous extracellular matrix (ECM) and endogenous growth factors.

When the material (A) is made of a non-bioabsorbable material having the dissolution rate as described above, the material (A) is prevented from deforming during placement in the body and easily forms an encapsulating membrane of the desired shape. The material (A) having the dissolution rate as described above also prevents the non-bioabsorbable material from partially remaining in the body and from being unable to be retrieved when the retrieval of the non-bioabsorbable material is needed.

The material (A) preferably satisfies both of the dissolution rate and the density as described above. The material (A) satisfying both of the dissolution rate and the density easily and efficiently activates the implantation site.

The degree of swelling of the material (A) is determined after immersion of the material (A) in physiological saline at 37°C for 60 minutes and may be selected in accordance with the type of material (A) or other factors. The degree of swelling of the material (A) calculated on mass basis may be selected from, for example, the range of about 200-fold or less, e.g., 150-fold or less, 120-fold or less, 100-fold or less or 80-fold or less, or may be 70-fold or less, e.g., 65-fold or less, preferably 60-fold or less, e.g., 55-fold or less, more preferably about 50-fold or less, in particular, 45-fold or less, e.g., 40-fold or less, 35-fold or less, 32-fold or less, 30-fold or less, 28-fold or less, 25-fold or less, 22-fold or less, 20-fold or less, 18-fold or less, 15-fold or less, 12-fold or less or 10-fold or less.

The lower limit of the degree of swelling calculated on mass basis, may be, for example, 1-fold (substantially no swelling), 1.5-fold, 2-fold, 3-fold, 5-fold, 6-fold, 7-fold, 8-fold, 8.5-fold, 9-fold, etc.

Especially when the material (A) is a bioabsorbable material, the degree of swelling may not be too small but adequately large, and may be, for example, 6-fold or more, 7-fold or more, 8-fold or more, 8.5-fold or more, 9-fold or more, 6 to 200-fold, 7 to 100-fold, 8 to 60-fold, etc.

When the material (A) is made of a bioabsorbable material as described later, the lower limit of the degree of swelling is a finite value in most cases. When the material (A) is made of a non-bioabsorbable material, the degree of swelling may be 1-fold or a small value in most cases.

The ratio of X/Y of the material (A) is defined by X and Y, wherein X is a density (mg/cm³) of the material (A) and Y is a degree of swelling of the material (A) expressed in terms of fold increase (fold increase by mass) calculated on mass basis after immersion of the material (A) in physiological saline at 37°C for 60 minutes. The ratio of X/Y may be selected from, for example, a range of about 0.01 or more, e.g., 0.02 or more, 0.03 or more, 0.05 or more, 0.08 or more, 0.1 or more, 0.12 or more, 0.15 or more, 0.18 or more, or 0.2 or more, or may be 0.21 or more, e.g., 0.22 or more, 0.23 or more, 0.24 or more, 0.25 or more, 0.28 or more, or 0.3 or more, preferably 0.4 or more, e.g., 0.5 or more, 0.6 or more, 0.7 or more, 0.8 or more, or 0.9 or more, more preferably 1 or more, e.g.,1.5 or more, or may be 2 or more, e.g., 2.5 or more, 3 or more, 3.5 or more, 4 or more, 4.5 or more, 5 or more, 5.5 or more, 6 or more, etc.

The upper limit of the ratio of X/Y may be, for example, 3000, 2500, 2000, 1500, 1200, 1000, 500, 400, 300, 200, 150, 120, 100, 90, 80, 70, 60, 50, 40, 35, 30, 20, 18, 15, 12, 10, etc., although the upper limit of the ratio of X/Y will depend on the type of material (A) or other factors.

Especially when the material (A) is a bioabsorbable material, the ratio of X/Y may not be too large but a rather small value, and may be, for example, 150 or less, 100 or less, 80 or less, 50 or less, 30 or less, 20 or less, 0.1 to 150, 0.2 to 120, 0.21 to 100, 0.25 to 50, etc.

When the material (A) has the degree of swelling and the ratio of X/Y as described above, the material (A) will retain a high density as exemplified above after the device is implanted. Such a material (A) will exhibit good handling during the implantation procedure and will be easily arranged or located at the targeted site and will promote the activation of the targeted site.

The material (A) may be, but is not limited to, a polymer or a resin.

The material (A) may be a bioabsorbable material or a non-bioabsorbable material.

The bioabsorbable material or the bioabsorbable polymer may be, for example, a protein, such as a gelatin, a collagen or a collagen peptide; a polysaccharide or its derivative, such as a mucopolysaccharide, e.g., hyaluronic acid or heparin, alginic acid, chitin, chitosan, starch, dextran, etc.; or an aliphatic polyester, such as polyglycolic acid, polylactic acid, a glycolic acid/lactic acid copolymer, poly-β-hydroxybutyrate, etc.

The bioabsorbable material may be any bioabsorbable material independently of whether the bioabsorbable material is actually absorbed in the body at the time of use of the device of the invention.

The non-bioabsorbable material may be the material as described later, e.g., a silicone resin, a polyvinyl alcohol resin, etc.

The material (A) may preferably contain the bioabsorbable material (A1).

When an encapsulating membrane is formed, the material (A) containing the bioabsorbable material (A1) is absorbed in the body and used to form part of the encapsulating membrane. Probably in this manner, an encapsulating membrane with an adequate thickness is easily formed.

The material (A) also tends to attract effector cells that secrete growth factors, and the attracted effector cells tend to accumulate in the material (A), which contributes to the effects of increasing the amount of endogenous extracellular matrix (ECM) and the levels of endogenous growth factors or of promoting the secretion of endogenous extracellular matrix (ECM) and endogenous growth factors.

The amount of the bioabsorbable material (A1) in the material (A) may be 10% by mass or more, e.g., 20% by mass or more, preferably 30% by mass or more, e.g., 40% by mass or more, more preferably 50% by mass or more, e.g., 60% by mass or more, or may be 70% by mass or more, e.g., 80% by mass or more, 90% by mass or more, 95% by mass or more, or 100% by mass. The material (A) may be made substantially of the bioabsorbable materials (A1) only.

The bioabsorbable material (A1) is preferably a protein, and is particularly preferably a gelatin. The material (A) or the bioabsorbable material (A1) contains at least a gelatin.

The gelatin serving as the bioabsorbable material (A1) will be described below.

The gelatin may be any type of gelatin or may be derived from any animal, such as fish, bovines or pigs, or may be a recombinant, e.g., a recombinant gelatin, a human recombinant gelatin, etc.

The gelatin may be produced by any method, including, e.g., alkaline treatment or acid treatment, etc.

The isoelectric point, molecular weight, molecular weight distribution, viscosity, jelly strength, etc., of the gelatin are not limited to particular ones and may be selected as appropriate.

The gelatin may be modified or derivatized. For example, a hydrophobic group, e.g., a hydrocarbon group, such as an alkyl group, may be introduced in the gelatin. For example, the amino groups of the gelatin may be acylated. For example, the amino groups of the gelatin may be subjected to alkanoylation such as hexanoylation and dodecanoylation. The gelatin may not be modified or derivatized.

The modifications or derivatizations may be introduced into any site of the gelatin molecule, and may be introduced into a molecular terminal or a side chain, etc. Such modifications or derivatizations are usually introduced to the extent or degree that the bioabsorbability of the gelatin is not deteriorated before and after the modifications or derivatizations.

A gelatin suitable for use in inhibition of inflammation or swelling at the implantation site includes a gelatin without any modifications or chemical modifications; an underivatized gelatin, e.g., a gelatin without any introduction of hydrophobic groups, etc.; a gelatin with a small amount of modifications or chemical modifications; or a gelatin with a low degree of derivatization, e.g., a gelatin with introduction of a small amount of hydrophobic groups, etc., e.g., a gelatin in which about 20 mol% or less, e.g., 10 mol% or less, 5 mol% or less, or 3 mol% or less of the total amino groups is modified or derivatized.

The material (A) containing the gelatin may typically be biocompatible.

The material (A) containing the gelatin serving as the bioabsorbable material (A1) may further contain an additional bioabsorbable material as needed.

Such an additional material may also typically be biocompatible.

The additional material serving as a bioabsorbable material includes the materials exemplified above excluding the gelatin, and may be, for example, a protein or peptide, such as a collagen or a collagen peptide; a polysaccharide or its derivative, such as a mucopolysaccharide, e.g., hyaluronic acid or heparin, alginic acid, chitin, chitosan, starch, dextran, etc.; or an aliphatic polyester, such as polyglycolic acid, polylactic acid, a glycolic acid/lactic acid copolymer, poly-β-hydroxybutyrate, etc.

These additional materials may be used alone or in combination of two or more types.

When the device of the invention contains the additional material serving as a bioabsorbable material, the amount of the gelatin relative to the amount of the bioabsorbable material or the bioabsorbable polymer may be, for example, 30% by mass or more, 50% by mass or more, 70% by mass or more, 80% by mass or more, 90% by mass or more, 95% by mass or more, etc., although the amount of the gelatin will depend on the type of additional material or other factors.

The bioabsorbable material (A1), in particular, the gelatin, or the bioabsorbable material (A1) containing the gelatin, or at least the gelatin contained in the bioabsorbable material (A1), is preferably subjected to crosslinking.

Crosslinking treatment enhances the strength of the bioabsorbable material and promotes the biological absorption of the material (A) or the bioabsorbable material over a predetermined period of time, e.g., over one week to three months. Probably due to the enhancement of the strength of the bioabsorbable material and the promotion of the biological absorption of the material (A) or the bioabsorbable material, crosslinking treatment on the bioabsorbable material is advantageous for the activation of the implantation site, in particular, formation of an encapsulating membrane or a uniform, encapsulating membrane, etc.

The crosslinking method may be, but is not limited to, crosslinking using a chemical reagent, e.g., crosslinking using a crosslinking agent, e.g., an aldehyde such as formaldehyde or glutaraldehyde, or a carbodiimide; crosslinking by heating, such as thermal dehydration crosslinking or thermal crosslinking; enzymatic crosslinking; crosslinking by energy rays, such as optical crosslinking, ultraviolet rays, electron rays or radioactive rays; physical crosslinking via, e.g., hydrogen bonds, hydrophobic interactions, ionic interactions, etc.; etc. Preferred among these is crosslinking by heating, such as thermal dehydration crosslinking etc.

The material (A), the device or the bioabsorbable material (A1) may contain an additional component. The additional component may be, for example, but is not limited to, a cell culture component, such as an alkali metal, an alkaline earth metal, a halogen, glucose, or a physiologically active substance, etc. as described later.

The additional component may be an angiogenic component, or a component capable of contributing to angiogenesis.

The angiogenic component or a component capable of promoting angiogenesis may be any component that contributes to or promotes angiogenesis. Examples of such a component include growth factors or cell growth factors, such as vascular endothelial growth factor (VEGF), fibroblast growth factor (FGF), hepatocyte growth factor (HGF), epidermal growth factor (EGF), insulin-like growth factor (IGF) (e.g., IGF-1, IGF-2, etc.), platelet-derived growth factor (PDGF), placental growth factor (PIGF) and insulin; and cells, such as cells capable of secreting growth factors or cell growth factors.

Examples of the cells include stem cells such as mesenchymal stem cells (MSCs), including, for example, adipose-derived stem cells (ADSCs), marrow-derived stem cells (BMSCs), placenta-derived stem cells, and umbilical cord-derived stem cells.

The material (A), the device 1 or the bioabsorbable material (A1) may be substantially free of such an additional component, e.g., an angiogenic component, and in particular, may be substantially free of growth factors.

The device of the invention may be capable of activating the implantation site as described above. Therefore, the activation of the implantation site can be achieved without inducing angiogenesis by growth factors, or the activation of the implantation site can be achieved in a different manner from angiogenesis by growth factors.

Use or administration of exogenous growth factors also differs from use of cells (ADSCs) etc. in that administration of exogenous growth factors may induce damage such as bleeding, inflammation and accumulation of blood and exudates due to angiogenesis or excessive or extreme angiogenesis. Such damage may also reduce the functions and/or the implantation performance of a cell-containing device as described later. However, growth factors are not used in the invention, and such damage can efficiently be reduced or prevented.

The material (A) or the bioabsorbable material (A1) may be in the form of a gel, a gelatinous mass, etc., or may have a porous structure or be porous.

The material (A) may typically have an appropriate shape or formed into an appropriate shape.

The form or shape of the material (A) may be selected depending on the desired physical property values, e.g., the density, as described above, and may be, for example, but is not limited to, particles or granules, a film or a sheet, a pillar such as a cylinder or a prism, a bar, a tube, etc.

The dimension or size of the material (A) can be selected as appropriate for the implantation site or its size and is not limited to a particular one. For example, the thickness of the material (A) may be 0.01 mm or more, e.g., 0.03 mm or more, preferably 0.05 mm or more, e.g., 0.1 to 50 mm, more preferably 0.15 mm or more, e.g., 0.2 to 10 mm, or may be 0.3 mm or more, e.g., 0.5 to 5 mm, 0.7 to 2 mm, 0.5 to 2 mm, 0.7 to 1.5 mm, etc.

### Additional material

The device of the invention comprises the material (A), and may be made of only the material (A) and may further contain an additional material.

The device of the invention may comprise, among others, at least a non-bioabsorbable material or member (may be called a non-bioabsorbable material (B), a material (B), etc.) as the additional material or member.

When the material (A) is made of a non-bioabsorbable material, the device of the invention exhibits various effects. For example, the device of the invention rarely develops adhesions to the surrounding tissue, and the device of the invention is easily retrieved and has other advantages.

When the material (A) is a non-bioabsorbable material, the device of the invention, in many cases, does not contain an additional material or an additional non-bioabsorbable material, or the device of the invention contains a non-bioabsorbable material as the material (A).

The device of the invention may be made or formed of the bioabsorbable material (A1) and the non-bioabsorbable material (B), and the bioabsorbable material (A1) may be the material (A) containing the bioabsorbable material (A1). Even when the bioabsorbable material (A1) is absorbed or dissolved in the body after implantation or placement of the device into the body, the non-bioabsorbable material remains in the body. The non-bioabsorbable material may serve as a spacer in the device, or may have a spacer-like function.

After the implanted device or at least the non-bioabsorbable material is retrieved, or after a portion of the device that has not been absorbed in the body, e.g., the non-bioabsorbable material, is retrieved while part or all of the bioabsorbable material is absorbed, the non-bioabsorbable material that is capable of remaining in the body forms a pocket or a space at the implantation site.

Such a pocket can serve as an indicator of the location of the implantation site that has been activated with the formation of an encapsulating membrane etc. and also serves as a pocket (an implantation pocket) into which a cell-containing device (described later) is subsequently implanted. The pocket is thus particularly useful for implantation of another device etc.

The non-bioabsorbable material will be described in detail below.

### Non-bioabsorbable material

The non-bioabsorbable material (B) typically contains or is made or formed of a non-bioabsorbable polymer.

Examples of the non-bioabsorbable polymer include, for example, silicone resins; polyvinyl alcohol resins; polyvinyl acetal resins; polyurethane resins; fluororesins, such as polytetrafluoroethylene and perfluoroalkoxyalkane; olefin resins, such as polyethylene resins and polypropylene resins; polyacrylamide resins; polyester resins, such as polyethylene terephthalate; polyacrylonitrile resins; polystyrene butadiene copolymer resins; polysulfone resins; cellulose resins, such as cellulose ethers, such as carboxymethyl cellulose and hydroxypropyl cellulose; and polyoxyalkylene resins, such as a resin containing ethylene oxide or propylene oxide as a polymerization component.

The non-bioabsorbable material or the non-bioabsorbable polymer is not absorbed in the body but may typically be biocompatible or a biocompatible polymer. The non-bioabsorbable material or the non-bioabsorbable polymer may be a hydrophilic polymer or a water-soluble polymer. The hydrophilic polymer or the water-soluble polymer contained in the device or the non-bioabsorbable material may typically be converted into a water-insoluble form, e.g., a gel, by crosslinking and/or chemical modifications or other techniques.

The non-bioabsorbable material preferably rarely develops adhesions to the implantation site or the surrounding tissue, i.e., preferably has an adhesion preventive ability. The adhesion preventive ability refers to, for example, the ability to prevent or inhibit the development of adhesions between the device and the surrounding tissue or the encapsulating membrane formed when the device is placed at an implantation site.

A silicone resin, a polyvinyl alcohol resin and others are preferred among the polymers as exemplified above.

A suitable polyvinyl alcohol resin (a polyvinyl alcohol resin (A)) may be a resin as described later, and may be, for example, a modified polyvinyl alcohol resin having an active carbonyl group (A1), a polyvinyl alcohol resin having a triad syndiotacticity of 32 to 40% (A2), a polyvinyl alcohol resin having a degree of saponification of 97 mol% or more (A3), etc.

A preferred embodiment etc. of such a polyvinyl alcohol resin may be the same as those described later.

The device of the invention containing such a non-bioabsorbable material develops few adhesions to the surrounding tissue, and at the time of retrieval, rarely causes damage to the implantation site, the encapsulating membrane formed, the surrounding tissue or a vascular bed at the implantation site, etc. In this manner, the device of the invention is capable of efficiently preventing or reducing bleeding, inflammation (induction of inflammation) or release of exudates from the implantation site, and due to this, the material (A) fully exhibits its functions.

The dissolution rate (the dissolution percentage or the residual percentage) of the non-bioabsorbable material as determined after immersion of the non-bioabsorbable material in physiological saline at 37°C for 24 hours may be, but is not limited to, for example, 5% by mass or less, preferably 3% by mass or less, more preferably 1% by mass or less, or may be 0% by mass (no substantial dissolution).

The degree of swelling of the non-bioabsorbable material calculated on mass basis after immersion of the non-bioabsorbable material in physiological saline at 37°C for 60 minutes may be, for example, 3-fold or less, 2-fold or less, 1.5-fold or less, etc., or may be 1-fold (no substantial swelling), etc.

The volume change of the non-bioabsorbable material as determined after immersion of the non-bioabsorbable material in physiological saline at 37°C for 60 minutes may be, for example, but is not limited to, 300% or less, e.g., 200% or less, 150% or less, 120% or less, preferably 100% or less, e.g., 80% or less, 70% or less, 60% or less, or may be 50% or less, e.g., 40% or less, 30% or less, 20% or less, 10% or less, 0% (no substantial volume change), etc.

The non-bioabsorbable material having the dissolution rate, the degree of swelling and the volume change as described above easily retains its shape when the device is implanted into the body, and easily produces an implantation pocket with the desired size and/or shape.

The non-bioabsorbable material may typically have an appropriate shape or formed into an appropriate shape.

The form or shape of the non-bioabsorbable material or member may be, for example, but is not limited to, particles or granules, a film or a sheet, a pillar such as a cylinder or a prism, a bar, a tube, etc.

The non-bioabsorbable material may be in the form of fibers, a woven fabric or a non-woven fabric, or may form a woven fabric or a non-woven fabric.

The non-bioabsorbable material may be in the form of a gel, a gum, etc., or may have a porous structure or a sponge structure or a network structure.

For example, the polyvinyl alcohol resin serving as the non-bioabsorbable material may be in the form of a gel, a sponge, etc. A preferred embodiment or physical properties etc. of the polyvinyl alcohol resin serving as the non-bioabsorbable material may be the same as those described later.

The dimension or size of the non-bioabsorbable material can be selected as appropriate for the implantation site or its size and is not limited to a particular one.

For example, the non-bioabsorbable material or a bulky non-bioabsorbable material may have a thickness of 0.01 mm or more, e.g., 0.03 mm or more, preferably 0.05 mm or more, e.g., 0.1 to 50 mm, more preferably 0.15 mm or more, e.g., 0.2 to 10 mm, particularly preferably 0.3 mm or more, e.g., 0.5 to 5 mm or 0.7 to 2 mm, etc.

The non-bioabsorbable material or the non-bioabsorbable polymer may be a commercially available product or may be produced or synthesized by a conventional method, depending on its type or form or its configuration in the device of the invention, etc., but is not limited to a particular one.

For example, the silicone resin serving as the non-bioabsorbable material may be a commercially available silicone rubber sheet or a synthesized silicone rubber sheet.

Alternatively, the polyvinyl alcohol resin serving as the non-bioabsorbable material may be, for example, a commercially available polyvinyl alcohol resin or a synthesized polyvinyl alcohol resin that is formed into a gel, in particular, an aqueous gel or a hydrogel, or a sponge, etc. by a conventional method.

For example, the polyvinyl alcohol resin may be gelled in accordance with the method as described later, e.g., using a crosslinking agent etc., depending on the type of the polyvinyl alcohol resin used. A preferred embodiment of such a polyvinyl alcohol resin may be the same as those described later.

The non-bioabsorbable material, regardless of a commercially available product or a synthesized product, may be formed into the desired shape as appropriate, if needed, in accordance with the shape of the implantation site etc. For example, a predetermined size and/or shape is punched out from the non-bioabsorbable material.

### Additional component

The non-bioabsorbable material or the non-bioabsorbable member may contain an additional component. The additional component is not limited to a particular one, and may be those exemplified in the section of the material (A).

The non-bioabsorbable material or the non-bioabsorbable polymer may be substantially free of such an additional component, e.g., an angiogenic component, and in particular, may be substantially free of growth factors due to the same reasons as described above.

### Device

The device of the invention comprises at least the material (A) and may further comprise an additional material or an additional member as described above.

The device, the material (A), or an additional material, e.g., a non-bioabsorbable material (B), may be substantially free of growth factors, as described above.

The material (A) in the device may typically form at least part of a surface of the device, or may be exposed on a surface of the device.

The material (A) may cover 10% or more, e.g., 20% or more, of the surface or surface area of the device, preferably, may cover 30% or more, e.g., 40% or more, of the surface or surface area of the device; more preferably, may cover 50% or more, e.g., 60% or more, of the surface or surface area of the device; or may cover 70% or more, e.g., 80% or more, 90% or more, 95% or more, or (substantially) 100% of the surface or surface area of the device.

When the device contains an additional material, the material (A) and the additional material, in particular, the non-bioabsorbable material, are preferably arranged in such a spatial relationship or a configuration that the material (A) forms at least part of a surface of the device.

When the device contains an additional material, the material (A) may be in contact with or separated from the additional material in the device, but in most cases, the material (A) is typically in contact with the additional material.

Examples of the device with such a configuration include, for example, a device comprising the material (A) covering at least part of a surface of an additional material (the non-bioabsorbable material), for example, a device comprising a sheet-shaped or another shaped material (A) (stacked) on at least one side of a sheet-shaped or another shaped non-bioabsorbable material, in particular, on both sides or the front and back sides of a sheet-shaped or another shaped non-bioabsorbable material; or other devices.

The amount of the additional material (the non-bioabsorbable material) in the device relative to 100 parts by volume of the material (A) may, for example, be 1 part by volume or more, e.g., 5 to 5000 parts by volume; may preferably be 10 parts by volume or more, e.g., 15 to 3000 parts by volume; may more preferably be 20 parts by volume or more, e.g., 25 to 1000 parts by volume; may be 30 parts by volume or more, e.g., 35 parts by volume or more, 40 parts by volume or more, 45 parts by volume or more, etc.; or may be 800 parts by volume or less, e.g., 500 parts by volume or less, 400 parts by volume or less, 300 parts by volume or less, 200 parts by volume or less, 150 parts by volume or less, etc.

When the additional material is contained in such an amount relative to that of the material (A) in the device, a good balance is achieved between the activation (formation of an encapsulating membrane etc.) of the implantation site and the formation of an implantation pocket.

The material (A) and the additional material in the device may typically be fixed to each other, or fixed to each other so that they are integrated together. Typically, the material (A) may be fixed to the additional material (the non-bioabsorbable material), or the material (A) and the additional material (the non-bioabsorbable material) may be fixed to each other. When the material (A) is fixed to the non-bioabsorbable material, the location of the site of activation (the site of formation of an encapsulating membrane etc.) can be easily and efficiently matched with, corresponded to or defined relative to the location of an implantation pocket.

The fixing method may be, for example, but is not limited to, suturing, e.g., suturing with a suture, etc.; sticking, e.g., sticking or pasting with medical glue; etc. A suitable fixing method among these is suturing. The suture and the glue may be biocompatible and/or bioabsorbable or biodegradable.

The material (A) and/or the additional material (the non-bioabsorbable material) may be allowed to swell in water or a given liquid medium selected depending on the type of materials. In cases where the materials are allowed to swell in water or a given liquid medium, the materials may be combined before or after the materials are allowed to swell in water or a given liquid medium.

### Applications etc.

The device, material or member of the invention can be implanted, in particular, can be implanted in a living body, or can be used for implantation or can be used for implantation of another device etc.

By implanting or placing the device of the invention, the implantation site can be activated.

In particular, by implanting the device of the invention, the following may be induced:
(1) formation of an encapsulating membrane,
(2) increase of the amount of extracellular matrix, or promotion of secretion of extracellular matrix,
(3) increase of the level of a growth factor, or promotion of secretion of a growth factor, etc.

Due to these effects, the device, material or member of the invention is suitable for use as a device for implantation of another device etc., an implantation device, an implantation material or an implantation member. Such a device capable of activating the implantation site can be used for at least one of the applications as described above.

The device of the invention, in particular, the device comprising the bioabsorbable material (A1) as the material (A), is generally capable of forming at least an encapsulating membrane, and can be used as a device at least for (1) formation of an encapsulating membrane.

The formation of an encapsulating membrane as described in the above (1) can be identified visually or other means by comparing the implantation site before and after the implantation. The encapsulating membrane is typically formed at the implantation site of the device or the bioabsorbable material or its neighboring site or periphery. The encapsulating membrane may be made of at least the bioabsorbable material of the device, or the bioabsorbable material that is absorbed in the body.

The extracellular matrix as described in the above (2) may be, for example, a collagen, such as collagen III or collagen IV; a laminin; etc.

The extracellular matrix may be a single type or a combination of two or more types.

The device of the invention is capable of increasing the amount of ECM or promoting the secretion of ECM to supply ECM. Probably in this manner, the device of the invention may further advantageously contribute to the activation and may inhibit or prevent the reduction in the functions of the cell-containing device described later, etc.

Increase of the amount of extracellular matrix or promotion of secretion of extracellular matrix can be determined by, for example, immunohistochemical assay.

Immunohistochemical assay will be briefly described below. Cells or a cell-containing device is implanted and then the surrounding tissue is harvested. The tissue is fixed in 4% paraformaldehyde and embedded in paraffin. An antibody suitable for immunohistochemical staining includes, for example, anti-collagen III (ab7778; Abcam), anti-collagen IV (ab6586; Abcam) and anti-laminin (ab11575; Abcam) antibodies. A suitable secondary antibody is EnVision+ System HRP-conjugated polymer rabbit antibody (4003; DAKO). Increase of the amount of extracellular matrix or promotion of secretion of extracellular matrix can be evaluated by determining the positive percentage of collagen III, collagen IV and laminin staining in the surrounding tissue.

Examples of the growth factor or cell growth factor as described in the above (3) include epidermal growth factors (EGF); insulin-like growth factors (IGF) such as IGF-1 or IGF-2; fibroblast growth factors (FGF) such as FGF12; hepatocyte growth factors (HGF); transforming growth factors (TGF) such as TGF-β1; and platelet-derived growth factors (PDGF) such as PDGF-A or PDGF-B.

These growth factors may be used alone or in combination of two or more.

The device of the invention may be capable of inducing a high-level increase of IGF-2 or promoting the secretion of a high level of IGF-2, among others.

Another factor that inhibits the activation would be related to induction of apoptosis by hypoxic environment, which is typically found in a subcutaneous site, etc. The device of the invention is capable of increasing the level of a growth factor or promoting the secretion of a growth factor, e.g., IGF-2 etc., thereby inhibiting apoptosis. Probably in this manner, the device of the invention may further advantageously contribute to the activation, and may inhibit or prevent the reduction in the functions of the cell-containing device.

Growth factors may tend to contribute to induction of angiogenesis. The device of the invention is capable of increasing the level of a growth factor, and may contribute to efficient activation and may allow the cell-containing device to efficiently exhibit its functions.

The device of the invention may also be capable of increasing the amount of or promoting the secretion of (4) a component other than extracellular matrix or a growth factor. Such a component other than extracellular matrix or a growth factor includes, for example, cell adhesion molecules, such as N-cadherin, ICAM-1 or VCAM-1; inducers, such as epithelial-mesenchymal transition inducers, such as c-MET, or hypoxia inducible factor 1α subunit (HIF-1α); angiogenesis inhibitors, such as vasohibin 1; CD31; versican; thrombospondin (TSP) 2; etc.

Increase of the level of a growth factor etc., or promotion of secretion of a growth factor etc. can be determined by, for example, real-time PCR.

Real-time PCR will be briefly described below. Cells or a cell-containing device is implanted and then the surrounding tissue is harvested. RNA is extracted from the tissue. Relative gene expression is determined using, for example, TaqMan Array 96-well FAST plates (4413257; Applied Biosystems). TaqMan Array contains 46 target genes and 2 endogenous control gene candidates. Samples are analyzed using StepOnePlus Real-Time polymerase chain reaction (PCR) system (Applied Biosystems) under amplification conditions of 50°C for 2 min, 95°C for 20 sec, followed by 40 cycles of 95°C for 1 sec and 60°C for 20 sec. The results are analyzed by Expression Suite Software ver. 1.3 (Applied Biosystems). Relative quantification (RQ) is performed by the comparative CT method to evaluate increase of the level of growth factors, or promotion of secretion of growth factors. 18S is used as a housekeeping gene.

The extracellular matrix and the growth factor as well as the component other than extracellular matrix or a growth factor are typically produced at the implantation site of the device or the bioabsorbable material or its neighboring site or periphery, and may be produced in or within the encapsulating membrane.

The device of the invention may achieve any one of the above (1) to (3) and (4), or may achieve a combination of any one of the above (1) to (3) and (4). When a combination of any one of the above (1) to (3) and (4) as well as angiogenesis as described later are achieved by the device of the invention, various factors in combination exhibit various effects and further efficiently activate the implantation site.

The device of the invention can typically be used as a device for implantation as described above.

In particular, the device of the present invention can be implanted or placed in the body of an animal, including a human.

By implanting or placing the device of the invention, the implantation site or the placement site can be activated. When the device of the invention comprises the bioabsorbable material (A1) as the material (A) or other cases, such activation may be accompanied by (1) formation of an encapsulating membrane, (2) increase of the amount of extracellular matrix, or promotion of secretion of extracellular matrix, and (3) increase of the level of a growth factor, or promotion of secretion of a growth factor, as described above.

The device of the invention may be capable of increasing the amount of or promoting the secretion of a component other than extracellular matrix or a growth factor.

The device of the invention may be implanted or placed to induce angiogenesis or formation of new blood vessels. The device of the invention is capable of achieving the activation as described above without exogenous administration of growth factors as described above. As the activation is induced, for example, the level of an endogenous growth factor etc. is increased or the secretion of an endogenous growth factor etc. is promoted by the device of the invention, angiogenesis or mild angiogenesis may occur. Such angiogenesis together with the formation of an encapsulating membrane etc. may activate the implantation site.

The degree of angiogenesis may be examined by, for example, immunohistochemical assay, computerized tomography (CT) angiography, or other methods.

For immunohistochemical assay, the device of the invention is implanted and then the surrounding tissue is harvested. The tissue is fixed in 4% paraformaldehyde and embedded in paraffin. A suitable immunohistochemical staining antibody is, for example, an anti-von Willebrand factor (vWF) antibody (ab7356; Merck Millipore). A suitable secondary antibody is EnVision+ System HRP-conjugated polymer rabbit antibody (4003; DAKO). The number of vWF-positive cells or the number of the vWF-positive cells in the stromal region is counted to assess the degree of angiogenesis.

For computerized tomography (CT) angiography, for example, ExiTron nano 12000 is suitable for use, which is an alkaline earth metal-based nanoparticulate contrast agent specifically formulated for CT of animals. The nanoparticulate contrast agent is intravenously injected via the tail vein. Angiography is performed using an X-ray CT scanner for experimental animals (Latheta LCT-200; Hitachi) etc. Angiogenesis can be assessed by calculating the blood vessel volume in the surrounding tissue of cells or a cell-containing device. To extract capillary blood vessels, the CT number for analyzing the vascular region is preferably defined as 100 or more.

The implantation according to the invention is performed using at least the device of the invention, and the device of the invention may be implanted as needed together with another device, for example, together with a device containing a non-bioabsorbable material, such as a polyvinyl alcohol resin or the resin as described later, a device containing a non-bioabsorbable material further containing cells (such as ADSCs) that secrete a growth factor.

The implantation site may be, for example, but is not limited to, a subcutaneous site; a subfascial site; the surface of an organ, e.g., the surface of the liver, spleen or other organs; an intraperitoneal site, e.g., under the greater omentum; under the mesentery; the peritoneal cavity; the inguinal region; etc. The implantation site may be muscular tissue, adipose tissue (adipocytes), etc. The implantation site is preferably a subcutaneous site or subcutaneous tissue.

Subcutaneous sites usually have few blood vessels, but the device of the present invention is capable of efficiently activating such a site with few blood vessels, independently of angiogenesis.

The device of the invention may be implanted or placed in any manner and may be implanted or placed in accordance with a conventional or known method. For example, instruments known to be used for implantation may be employed.

The subject for implantation of the device of the invention and optionally a cell-containing device as described later may be a human or a non-human animal, for example, a mammal such as a dog or a cat. The non-human animal may be a companion animal. A preferred subject is a human.

The duration of placement of the device of the invention may vary depending on the implantation site or other factors, but the implanted device is preferably left at the implantation site for, for example, one week or more, more preferably for 10 days to three months, and particularly preferably for two weeks to two months.

When the device of the invention is left in the implantation site for such a period of time, sufficient activation of the implantation site, including formation of an encapsulating membrane, etc., are easily achieved.

An encapsulating membrane containing the bioabsorbable material (A1) of the device or containing the material (A) containing the bioabsorbable material (A1) is usually formed by the implantation or placement of the device of the invention, as described above. After formation of the encapsulating membrane, the bioabsorbable material (A1) may be gradually absorbed in the body over time. The duration of placement of the device, or the duration of placement of the device until retrieval of the device (as described later) may be determined so that at least the encapsulating membrane containing the bioabsorbable material (A1), i.e., the encapsulating membrane containing the residual bioabsorbable material (A1), is retained at the implantation site.

The implanted device of the invention may be retrieved from the implantation site after a predetermined duration of placement has past. In most cases, when the device of the invention comprising the non-bioabsorbable material is implanted, for example, when the device of the invention comprising the non-bioabsorbable member as a material (A) is implanted, or when the device of the invention comprising the non-bioabsorbable member (B) is implanted, in particular, when the device of the invention comprising the material (A) containing the bioabsorbable material (A1) and comprising the non-bioabsorbable material (B) is implanted, at least the non-bioabsorbable material, typically, only the non-bioabsorbable material, is retrieved from the implantation site. The present invention also includes such a method for retrieving the non-bioabsorbable material.

In most cases, when the device of the invention or the material (A) containing the bioabsorbable material (A1) is placed in the body for the predetermined period of time, e.g., for one week to three months, part or all of the bioabsorbable material (A1) is typically absorbed in the body and forms an encapsulating membrane. However, the non-bioabsorbable material does not absorbed in the body but remains as it is at the implantation site. The non-bioabsorbable material needs to be retrieved when a cell-containing device etc. is subsequently implanted into the implantation site.

The retrieval of the device of the invention and/or the non-bioabsorbable material is usually performed without causing bleeding, inflammation, rupture of existing blood vessels or newly formed blood vessels, damage to the encapsulating membrane or the formed encapsulating membrane, etc. If bleeding, inflammation, rupture of blood vessels or damage to the encapsulating membrane occurs, the blood or exudates may accumulate at the site from which the device and/or the non-bioabsorbable material has been retrieved.

The non-bioabsorbable material as described above, e.g., a silicone resin, a polyvinyl alcohol resin, etc., is selected for use in the present invention, and the duration of placement of the device is also selected as appropriate. Due to these materials and the duration of placement and/or other factors as well as less probability of development of adhesions to the implantation site, the device of the invention and/or the non-bioabsorbable material can usually be efficiently retrieved without causing bleeding, inflammation or rupture of blood vessels.

An implantation pocket is formed at the site from which the device of the invention and/or the non-bioabsorbable material has been retrieved. A cell-containing device etc. may subsequently be implanted into the implantation pocket as described later.

The implantation site of the device of the invention is activated as described above, and another device may subsequently be implanted into the implantation site. Implantation of a cell- or tissue-containing device as another device will be described in detail below.

### Cell- or tissue-containing device

The present invention also includes a method for implanting or placing a device comprising cells or tissue (a cell- or tissue-containing device; may also be called a cell-containing device or a device 2 below).

Implantation of the cell-containing device (the cell-containing component) may be performed in parallel to the implantation or placement of the device of the invention (the device 1 or the gelatin-containing device) or may be performed at the same time of the implantation or placement of the device of the invention (the device 1 or the gelatin-containing device). Alternatively, the implantation of the cell-containing device (the cell-containing component) may not be performed in parallel to the implantation or placement of the device of the invention (the device 1 or the gelatin-containing device) or may not be performed at the same time of the implantation or placement of the device of the invention (the device 1 or the gelatin-containing device).

When the implantation of the cell-containing device is performed in parallel to the implantation of the device 1, the device 1 may be implanted near or at the periphery of the cell-containing device, or may be implanted adjacent to the cell-containing device.

The cell-containing device may be implanted or placed at the site in which the device 1 has been implanted and subsequently the device 1 or at least the non-bioabsorbable material has been retrieved, i.e., at the implantation site or into the implantation pocket.

In either of the cases, when an encapsulating membrane is formed by the device 1, the cell-containing device is preferably implanted after the encapsulating membrane is formed or while the encapsulating membrane is retained or at the time when the encapsulating membrane is formed or retained, in particular, while the gelatin is contained or remains in the encapsulating membrane.

In other words, the device of the invention (the device 1) can be used as a device for use in combination with the cell-containing device, in particular, as a device to be implanted in the implantation site of the cell-containing device or the implantation site that is to be activated, e.g., the implantation site where an encapsulating membrane is to be formed, etc., or as a device to be implanted prior to implantation of the cell-containing device in the implantation site of the cell-containing device or the implantation site that is to be activated, e.g., the implantation site where an encapsulating membrane is to be formed, etc.

The device of the invention (the device 1) is capable of activating the implantation site of the cell-embedding device or the neighboring site or the periphery thereof as described above, thereby allowing the cell-containing device to efficiently exhibit its functions.

In this manner, situations can be avoided where living cells or tissue is implanted before the implantation site is activated, and consequently central necrosis and apoptosis may be efficiently inhibited or prevented, thereby allowing the cell-containing device to exhibit its functions. Replacement of the implanted cell-containing device is also easily performed when it is needed.

The device 1 containing a non-bioabsorbable material can be adapted to rarely develop adhesions to the surrounding tissue and to be retrieved (at least the non-bioabsorbable material can be retrieved) without causing bleeding, inflammation or rupture of blood vessels by selecting the type of non-bioabsorbable material etc., as described above. When the cell-containing device is implanted into such a site from which the device 1 has been retrieved, the cell-containing device can be easily exhibit its functions much more efficiently.

Implantation of the cell-containing device into such a site from which the device 1 has been implanted and subsequently retrieved is understood as meaning that the cell-containing device is efficiently implanted into an implantation site without bleeding, inflammation or rupture of blood vessels.

The inventors studied and found that, when the retrieval of the device 1 is accompanied by bleeding, inflammation and/or rupture of blood vessels, blood and/or exudates may easily accumulate between an implanted cell-containing device and the implantation site. The present invention facilitates the implantation or placement of a cell-containing device at an implantation site without being accompanied by bleeding, inflammation and/or rupture of blood vessels, and also activates the implantation site as described above, thereby allowing the cell-containing device to efficiently exhibit its functions.

Bleeding, inflammation and/or rupture of blood vessels, if occurs, at the implantation site can be visually observed, for example, before and after the implantation or retrieval or harvest of the device 1 or before the implantation of the cell-containing device.

The cell-containing device as described above may be cells or tissue itself, or embedded cells or tissue (may also be called the cell-embedding device below).

The cell-embedding device may be any cell-embedding device and may be, for example, but is not limited to, the cell-embedding device or the like described in Patent literature 1 or 2. The cell-embedding device will be described in detail below.

A biological composition (e.g., cells or tissue) contained in the cell-containing device is not particularly limited and may be selected as appropriate for the purpose of use.

The biological composition may be, for example, differentiated cells, stem cells or other types of cells derived from the ectoderm, mesoderm or endoderm.

The differentiated cells may be, for example, epidermal cells, smooth muscle cells, osteocytes, bone marrow cells, chondrocytes, skeletal myoblasts, pancreatic parenchymal cells, pancreatic islet cells, pancreatic endocrine cells, pancreatic exocrine cells, pancreatic ductal cells, liver cells (e.g., hepatocytes), thyroid cells, parathyroid cells, adrenal cells, pituitary cells, splenic cells, pinealocytes, renal cells (nephrocytes), spleen cells, anterior pituitary cells, somatotropic cells, dopamine-producing cells, blood cells, cardiac muscle cells, skeletal muscle cells, osteoblasts, nerve cells, pigment cells, adipocytes, etc. These cells may be cells isolated from a living body or may be cells differentiated from stem cells as described later.

The stem cells (e.g., iPS cells) and other types of cells that can be induced to differentiate may be implanted as it is in a living body, or incorporated in the device and implanted in a living body and then induced to differentiate in the living body. Alternatively, differentiated cells may be implanted in a living body or incorporated in the device.

The stem cells may be, but are not limited to, tissue-resident stem cells, such as epidermal stem cells, hair follicle stem cells, pancreatic stem cells, pancreatic progenitor cells, liver stem cells, neural stem cells, retinal stem cells, and hematopoietic stem cells; embryonic stem cells (ES cells); induced pluripotent stem cells (iPS cells); etc.

These cells are preferably from a mammal, such as a human, a monkey, a pig, a rat, a mouse, a dog, or cat, and are preferably capable of producing and/or secreting a physiologically active substance, such as a hormone or a protein useful for a living body, such as a patient. The type of cells to be implanted can be selected depending on the type of disease in the recipient, such as a patient, to undergo the implantation. When the cells are non-human cells, the cells may carry a human gene introduced thereinto for therapeutic purposes. Examples of the hormone useful for a living body include insulin, thyroid-stimulating hormone, thyroid hormones, parathyroid hormone, growth hormone, thyroxine, glucocorticoids, glucagon, estradiol, and testosterone. Specific examples of the protein useful for a living body include blood coagulation factors, complements, albumin, globulin, and various enzymes, such as metabolic enzymes or digestive enzymes, such as amylase, protease and lipase. Examples of other physiologically active substances include neurotransmitters, such as dopamine.

Specifically, the cells are more preferably pancreatic cells, in particular, pancreatic islet cells, hepatocytes, dopamine-producing cells, or stem cells or progenitor cells that give rise to these cells, and are further preferably pancreatic cells, in particular, pancreatic islet cells, or pancreatic progenitor cells or pancreatic stem cells. In an exemplary example, pancreatic islets or pancreatic cells, hepatocytes, stem/progenitor cells that give rise to these cells, etc. may be suitable for use.

The biological composition contained in the cell-containing device may be cells or living tissue established for laboratory use, or cells isolated from living tissue. The biological composition is preferably differentiated non-dividing cells. The isolation may be performed by any isolation method in accordance with a conventionally known method. The cells isolated from living tissue are desirably subjected to removal of pathogens, such as pathogenic viruses.

The amount of the biological composition contained in the cell-containing device may be adjusted as appropriate for the type of biological composition.

The dosage is determined by a physician considering the age, sex and symptoms of the patient, adverse side effects, and other factors, and is not limited to a specific amount. Typically, about one to ten devices may be implanted in an adult human. For example, a diabetic patient may receive the cell-containing device containing typically 1,000 to 1,000,000 IEQ/kg body weight islets, preferably 5,000 to 400,000 IEQ/kg body weight islets, and more preferably 10,000 to 20,000 IEQ/kg body weight islets (IEQ: international unit of the number of pancreatic islets; one IEQ is defined as the volume of a single islet of 150 µm in diameter).

The cell-embedding device is formed by embedding the biological composition in the device as described above. The cell-embedding device may be in any shape, and may be in a similar shape as that of the device 1, in particular, a similar shape as that of the non-bioabsorbable material contained in the device 1, and may, in particular, be in the same shape as that of the device 1, in particular, the same shape as that of the non-bioabsorbable material contained in the device 1. The cell-embedding device may thus be in the shape as exemplified above, including a disk, a sphere, a cylinder, an ellipsoid or other shapes, but is preferably in a disk shape.

The cell-embedding device, in particular, the device element thereof, may be made of any material, and may be made of a macromolecule (polymer), a metal, ceramics, etc.

The polymer used to produce the cell-embedding device is not limited to a particular polymer, and may be selected from, for example, structural proteins such as collagen, elastin and keratin; gelatin; glycosaminoglycans such as hyaluronic acid, chondroitin sulfate, keratan sulfate, dermatan sulfate, heparan sulfate and heparin; proteoglycans; cell adhesion molecules such as fibronectin, laminin, fibrinogen and vitronectin; fibrin; fibroin; sericin; alginic acid; chitosan; agarose; cellulose; cellulose derivatives such as cellulose nanofibers, carboxymethyl cellulose, and hydroxypropyl cellulose; synthetic polypeptides; polylactic acid; polyglycolic acid; polycaprolactone; polyethylene glycol; polypropylene glycol; 2-methacryloyloxyethyl phosphorylcholine; poly(meth)acrylic acid; polyacrylamide; poly-N-isopropylacrylamide; dextrin; silicone; polyurethane; fluororesins such as polytetrafluoroethylene and perfluoroalkoxyalkane; polyvinyl alcohol resins; and others.

The polymer may be a hydrophilic polymer or a water-soluble polymer. The hydrophilic polymer or the water-soluble polymer contained in the cell-embedding device may typically be converted into a water-insoluble form, e.g., a gel, by crosslinking or other techniques.

The polymer may be a single type or a combination of two or more types.

A polyvinyl alcohol resin is particularly preferred among the polymers as exemplified above. Accordingly, the polymer may contain at least a polyvinyl alcohol resin.

The polyvinyl alcohol resin will be described in detail below.

### Polyvinyl alcohol resin

The polyvinyl alcohol resin may typically be a saponified product of a polymer containing at least a vinyl ester monomer as a polymerization component. Such a polyvinyl alcohol resin may be any polyvinyl alcohol resin containing vinyl alcohol units, and may contain vinyl ester units or structural units derived from a vinyl ester monomer, e.g., structural units derived from a fatty acid vinyl ester, such as vinyl acetate units and vinyl pivalate units (described later) and/or may contain other units, e.g., structural units derived from an unsaturated monomer having an active carbonyl group (described later) or other structural units derived from other unsaturated monomers.

The viscosity of a 4% by mass aqueous solution of the polyvinyl alcohol resin (at 20°C) may be, for example, but is not limited to, 1 mPa·s or more, 2 mPa·s or more, 3 mPa.s or more, 5 mPa.s or more, 20 mPa.s or more, 30 mPa.s or more, 40 mPa.s or more, 50 mPa·s or more, etc., or may be, for example, 800 mPa·s or less, 500 mPa·s or less, 300 mPa·s or less, 200 mPa·s or less, 150 mPa·s or less, 100 mPa·s or less, 80 mPa·s or less, etc.

Typically, a polyvinyl alcohol resin having a viscosity of about 3 to 300 mPa·s as measured as a 4% by mass aqueous solution may be used as a suitable polyvinyl alcohol resin.

The viscosity of a 4% by mass aqueous solution of a polyvinyl alcohol resin may be measured in accordance with, for example, JIS K 6726.

The polyvinyl alcohol resin used in the invention can be selected in terms of its type, composition, etc., and may be, but is not limited to, a completely saponified polyvinyl alcohol resin (e.g., having a degree of saponification of 97 mol% or more), or a partially saponified polyvinyl alcohol resin (e.g., having a degree of saponification of less than 97 mol%).

The (average) degree of saponification may be measured in accordance with, for example, JIS K 6726.

In particular, a polyvinyl alcohol resin suitable as the polyvinyl alcohol resin (A) may be at least one selected from a modified polyvinyl alcohol resin having an active carbonyl group (A1), a polyvinyl alcohol resin having a triad syndiotacticity of 32 to 40% (A2), and a polyvinyl alcohol resin having a degree of saponification of 97 mol% or more (A3).

These resins (A1) to (A3) will be described in detail below.

### Modified polyvinyl alcohol resin having active carbonyl group

The modified polyvinyl alcohol resin having an active carbonyl group (simply called the modified PVA resin herein) may be, for example, a modified PVA copolymer produced by copolymerization of a fatty acid vinyl ester with an unsaturated monomer having an active carbonyl group followed by saponification of the resulting copolymer, or a post-modified PVA produced by directly contacting a conventionally produced PVA or modified PVA resin with a compound having an active carbonyl group, such as liquid diketene or diketene gas. A modified PVA copolymer is preferred for better stability and safety of the PVA resin and for better workability in the gelation process.

The fatty acid vinyl ester used in the production of the modified PVA copolymer may be, for example, but is not limited to, vinyl formate, vinyl acetate, vinyl propionate, vinyl pivalate, etc., and vinyl acetate is industrially preferred. These fatty acid vinyl esters may be subjected to a conventionally known polymerization method, such as bulk polymerization, solution polymerization, suspension polymerization, and emulsion polymerization. Solution polymerization using an alcoholic solvent, such as methanol, is industrially preferred.

The unsaturated monomer having an active carbonyl group may be, for example, but is not limited to, diacetone acrylamide, diacetone methacrylamide, diacetone acrylate, diacetone methacrylate, acetoacetoxy acrylamide, acetoacetoxy methacrylamide, etc. These unsaturated monomers may be used alone or in combination of two or more. Diacetone acrylamide is industrially preferred. The modified PVA copolymer is preferably diacetone acrylamide-modified PVA.

The copolymerization reaction between the fatty acid vinyl ester and the unsaturated monomer having an active carbonyl group according to the present invention may further include an additional unsaturated monomer capable of copolymerizing with the fatty acid vinyl ester and the unsaturated monomer having an active carbonyl group to the extent that the additional unsaturated monomer does not impair the effects of the invention.

The additional unsaturated monomer may be one or more unsaturated monomers selected from carboxyl group-containing unsaturated monomers, such as (meth)acrylic acid, maleic acid, maleic anhydride, fumaric acid, crotonic acid, itaconic acid, and undecylenic acid; unsaturated dibasic acid monoalkyl esters, such as monomethyl maleate and monomethyl itaconate; amide group-containing unsaturated monomers, such as acrylamide, dimethylacrylamide, dimethylaminoethylacrylamide, diethylacrylamide, dimethylaminopropylacrylamide, isopropylacrylamide, N-methylolacrylamide, and N-vinylacetamide; vinyl halides, such as vinyl chloride and vinyl fluoride; glycidyl group-containing unsaturated monomers, such as allyl glycidyl ether and glycidyl methacrylate; lactam group-containing unsaturated monomers including, e.g., N-vinylpyrrolidones such as N-vinyl-2-pyrrolidone and N-vinyl-alkylpyrrolidone (e.g., N-vinyl-mono- or di-C₁₋₄ alkyl-pyrrolidones, such as N-vinyl-3-propyl-2-pyrrolidone, N-vinyl-5-methyl-2-pyrrolidone, N-vinyl-5-ethyl-2-pyrrolidone, N-vinyl-5,5-dimethyl-2-pyrrolidone, and N-vinyl-3,5-dimethyl-2-pyrrolidone), N-allylpyrrolidones such as N-allyl-2-pyrrolidone, N-vinylpiperidones such as N-vinyl-2-piperidone and N-vinyl-alkylpiperidone (e.g., N-vinyl-mono- or di-C₁₋₄ alkyl-piperidones, such as N-vinyl-6-methyl-2-piperidone and N-vinyl-6-ethyl-2-piperidone), and N-vinylcaprolactams such as N-vinyl-ε-caprolactam and N-vinyl-alkylcaprolactam (e.g., N-vinyl-mono- or di-C₁₋₄ alkyl-caprolactams, such as N-vinyl-7-methyl-2-caprolactam and N-vinyl-7-ethyl-2-caprolactam); alkyl vinyl ethers such as C₁₋₂₀ alkyl vinyl ethers (e.g., methyl vinyl ether, n-propyl vinyl ether, i-propyl vinyl ether, n-butyl vinyl ether, i-butyl vinyl ether, t-butyl vinyl ether, lauryl vinyl ether, dodecyl vinyl ether, and stearyl vinyl ether); nitriles such as acrylonitrile and methacrylonitrile; hydroxy group-containing unsaturated monomers, including, e.g., C₁₋₂₀ monoalkyl allyl alcohols such as allyl alcohol and isopropenyl allyl alcohol, C₁₋₂₀ dialkyl allyl alcohols such as dimethyl allyl alcohol, and hydroxy C₁₋₂₀ alkyl vinyl ethers such as hydroxy ethyl vinyl ether and hydroxy butyl vinyl ether; acetyl group-containing unsaturated monomers, including, e.g., C₁₋₂₀ alkyl allyl acetates such as allyl acetate, dimethylallyl acetate, and isopropenylallyl acetate; (meth)acrylic acid esters, including, e.g., (meth)acrylic acid alkyl esters, such as (meth)acrylic acid C₁₋₂₀ alkyl esters such as methyl (meth)acrylate, ethyl (meth)acrylate, 2-ethylhexyl acrylate, and n-butyl acrylate; vinylsilanes such as trimethoxyvinylsilane, tributylvinylsilane, and diphenylmethylvinylsilane; polyoxyalkylene (meth)acrylates, such as polyoxyethylene (meth)acrylate and polyoxypropylene (meth)acrylate; polyoxyalkylene (meth)acrylamides, such as polyoxyethylene (meth)acrylamide and polyoxypropylene (meth)acrylamide; polyoxyalkylene vinyl ethers, such as polyoxyethylene vinyl ether and polyoxypropylene vinyl ether; polyoxyalkylene alkylvinyl ethers, such as polyoxyethylene allyl ether, polyoxypropylene allyl ether, polyoxyethylene butylvinyl ether, and polyoxypropylene butylvinyl ether; α-olefins such as ethylene, propylene, n-butene, and 1-hexene; butenes such as 3,4-dihydroxy-1-butene, 3,4-diacyloxy-1-butene, 3-acyloxy-4-hydroxy-1-butene, 4-acyloxy-3-hydroxy-1-butene, and 3,4-diacyloxy-2-methyl-1-butene; pentenes such as 4,5-dihydroxy-1-pentene, 4,5-diacyloxy-1-pentene, 4,5-dihydroxy-3-methyl-1-pentene, and 4,5-diacyloxy-3-methyl-1-pentene; hexenes such as 5,6-dihydroxy-1-hexene and 5,6-diacyloxy-1-hexene; unsaturated amine monomers, such as N,N-dimethylallylamine, N-allylpiperazine, 3-piperidine acrylic acid ethyl ester, 2-vinylpyridine, 4-vinylpyridine, 2-methyl-6-vinylpyridine, 5-ethyl-2-vinylpyridine, 5-butenylpyridine, 4-pentenylpyridine, and 2-(4-pyridyl)allyl alcohol; quaternary ammonium compound-containing unsaturated monomers, such as dimethylaminoethyl acrylate methyl chloride quaternary salt, N,N-dimethylaminopropyl acrylamide methyl chloride quaternary salt, and N,N-dimethylaminopropyl acrylamide methyl benzenesulfonate quaternary salt; aromatic unsaturated monomers such as styrene; sulfonic acid group-containing unsaturated monomers, such as 2-acrylamide-2-methylpropanesulfonic acid or its alkali metal salts, ammonium salts or organic amine salts, 2-acrylamide-1-methylpropanesulfonic acid or its alkali metal salts, ammonium salts or organic amine salts, 2-methacrylamide-2-methylpropanesulfonic acid or its alkali metal salts, ammonium salts or organic amine salts, vinyl sulfonic acid or its alkali metal salts, ammonium salts or organic amine salts, allyl sulfonic acid or its alkali metal salts, ammonium salts or organic amine salts, and methallyl sulfonic acid or its alkali metal salts, ammonium salts or organic amine salts; glycerol monoallyl ether; 2,3-diacetoxy-1-allyloxypropane; 2-acetoxy-1-allyloxy-3-hydroxypropane; 3-acetoxy-1-allyloxy-3-hydroxypropane; 3-acetoxy-1-allyloxy-2-hydroxypropane; glycerol monovinyl ether; glycerol monoisopropenyl ether; acryloyl morpholine; vinyl ethylene carbonate; vinylimidazole; and vinylcarbazole.

The amount of the additional unsaturated monomer may be, for example, but is not limited to, 10 mol or less relative to 100 mol of the vinyl ester monomers.

The produced modified PVA copolymer may be post-modified by acetalization, urethanation, etherification, grafting, phosphorylation, acetoacetylation, cationization, or other reactions in accordance with a known method to the extent that the effects of the present invention are not impaired.

A polymerization catalyst is used in the production of the modified PVA copolymer, and may typically be, but is not limited to, an azo compound or a peroxide.

An organic acid, such as tartaric acid, citric acid, and acetic acid, may be added to the polymerization reaction to prevent the hydrolysis of the fatty acid vinyl ester.

A polymerization terminator may be used to terminate the polymerization. The polymerization terminator may be, for example, but is not limited to, m-dinitrobenzene etc.

The shape of a polymerization vessel, the type of a polymerization agitator, the polymerization temperature, the pressure in the polymerization vessel, or other conditions in the copolymerization of the fatty acid vinyl ester and the unsaturated monomer having an active carbonyl group according to the present invention may be in accordance with a conventionally known method.

The copolymer of the fatty acid vinyl ester and the unsaturated monomer having an active carbonyl group may be saponified by any conventionally known method according to the present invention. For example, a conventionally known alcoholysis or hydrolysis is applicable using a basic catalyst, such as sodium hydroxide, potassium hydroxide or sodium methoxide, or an acidic catalyst, such as hydrochloric acid, sulfuric acid or p-toluenesulfonic acid.

The solvent used in the saponification reaction may be, for example, an alcohol such as methanol or ethanol; an ester such as methyl acetate; a ketone such as acetone or methyl ethyl ketone; an aromatic hydrocarbon such as benzene or toluene; tetrahydrofuran; etc. These solvents may be used alone or in combination of two or more. The temperature, duration, and other conditions of the saponification reaction are not particularly limited.

The drying, grinding or washing of the saponified product may be done by any methods and may be performed by a conventionally known method.

When the modified PVA resin is a PVA modified with the unsaturated monomer having an active carbonyl group (e.g., diacetone acrylamide), the amount of the unsaturated monomer units having an active carbonyl group (e.g., diacetone acrylamide units) contained in the modified PVA resin is, for example, 0.5 to 20 mol%, preferably 0.5 to 15 mol%, more preferably 1 to 12 mol%, and further preferably 2 to 10 mol% (e.g., 3 to 8 mol%) relative to the all the structural units in the modified PVA resin (relative to the total amount of the structural monomers).

The amount of the unsaturated monomer units having an active carbonyl group (e.g., diacetone acrylamide units) contained in the modified PVA resin is preferably 0.5 mol% or more, and the modified PVA resin containing such an amount of the unsaturated monomer units may have many reactive sites with a crosslinking agent and have sufficient strength (stress) as a cell-embedding device. The amount of the unsaturated monomer units contained in the modified PVA resin is preferably 20 mol% or less, and the modified PVA resin containing such an amount of the unsaturated monomer units may have improved solubility in water.

The degree of saponification of the modified PVA resin is preferably, but not limited to, 80 mol% or more (e.g., 80 to 99.9 mol%), more preferably 88 mol% or more (e.g., 88 to 99.9 mol%), and further preferably 95 mol% or more (e.g., 95 to 99.9 mol%).

The viscosity of the modified PVA resin may vary. A 4 mass% aqueous solution of the modified PVA resin (at 20°C) may have a viscosity of preferably 2 to 500 mPa·s, more preferably 3 to 300 mPa·s, and further preferably 5 to 200 mPa·s, e.g., 5 to 80 mPa·s, and may have a viscosity of typically 500 mPa·s or less, e.g., 20 to 500 mPa·s, 30 to 500 mPa·s, 40 to 500 mPa·s, 50 to 500 mPa·s, etc.; 300 mPa·s or less, e.g., 20 to 300 mPa·s, 30 to 300 mPa·s, 40 to 300 mPa·s, 50 to 300 mPa·s; 200 mPa·s or less, e.g., 20 to 200 mPa·s, 30 to 200 mPa·s, 40 to 200 mPa·s, 50 to 200 mPa·s; etc.

The degree of saponification and the viscosity of a 4 mass% aqueous solution may be measured in accordance with JIS K 6726.

### Polyvinyl alcohol resin having triad syndiotacticity of 32 to 40%

The polyvinyl alcohol resin may preferably be a polyvinyl alcohol resin having a triad syndiotacticity of 32 to 40% (simply also called the highly syndiotactic PVA resin herein).

The highly syndiotactic PVA resin preferably has a triad syndiotacticity of 32 to 40%, more preferably 33 to 39%, and further preferably 34 to 38%. The highly syndiotactic PVA resin having a triad syndiotacticity of 32% or more easily forms an aqueous gel, and the highly syndiotactic PVA resin having a triad syndiotacticity of 40% or less can easily form an aqueous gel.

The triad syndiotacticity can be determined from the peaks attributed to hydroxy groups in the proton NMR analysis of the highly syndiotactic PVA resin dissolved in deuterated dimethylsulfoxide (DMSO).

The highly syndiotactic PVA resin may be produced by any method that yields the highly syndiotactic PVA resin having a triad syndiotacticity of 32 to 40%. The highly syndiotactic PVA resin can easily be produced by saponifying a vinyl ester polymer prepared by a conventionally known method.

That is, the highly syndiotactic PVA resin is a saponified product of a vinyl ester polymer.

The vinyl ester polymer may be produced by any method that includes polymerization of vinyl ester monomers, and may be performed in accordance with a conventionally known method.

The shape of a polymerization vessel, the type of a polymerization agitator, the polymerization temperature, the pressure in the polymerization vessel, or other conditions may also be in accordance with a conventionally known method. The polymerization method may be selected from various conventionally known methods, including bulk polymerization, solution polymerization, suspension polymerization, emulsion polymerization, or other polymerization methods. For ease of controlling the degree of polymerization or performing a saponification reaction after polymerization, preferred polymerization methods are, but not limited to, solution polymerization using an alcohol as a solvent, or suspension polymerization using water or a combination of water and an alcohol as a dispersion medium.

Examples of the vinyl ester monomers include vinyl esters, such as fatty acid vinyl esters and non-fatty acid vinyl esters, e.g., vinyl formate, aromatic carboxylic acid vinyl esters, etc. For production of a PVA having a high syndiotacticity, the vinyl ester monomers may be C₃₋₁₅ fatty acid vinyl esters, e.g., linear or branched C₃₋₁₅ fatty acid vinyl esters, such as vinyl propionate, vinyl butyrate, and vinyl pivalate, and preferably C₃₋₁₀ fatty acid vinyl esters, such as linear or branched C₃₋₁₀ fatty acid vinyl esters; C₃₋₁₅ fatty acid vinyl esters having one or more substituents (e.g., a halogen group), such as vinyl trifluoroacetate and vinyl trichloroacetate; vinyl formate; etc. These vinyl esters can be used alone or in combination of two or more.

Specific examples of the method for producing the highly syndiotactic PVA include a method including homopolymerization or copolymerization of vinyl ester monomers having a bulky side chain, such as vinyl propionate, vinyl butyrate, and vinyl pivalate, followed by saponification of the product using an alkaline catalyst; and a method including homopolymerization or copolymerization of vinyl ester monomers having a high polarity, such as vinyl formate, vinyl trifluoroacetate, and vinyl trichloroacetate, followed by saponification of the product using an alkaline catalyst. Preferred methods include polymerization of vinyl pivalate followed by saponification of the product using an alkaline catalyst. An example of a production process of a PVA resin having a triad syndiotacticity of 37.1% will be described in Examples later. The triad syndiotacticity of the PVA resin can be reduced to less than 37.1% by, for example, adding vinyl acetate to the polymerization reaction of vinyl pivalate to give a vinyl pivalate-vinyl acetate copolymer, or by raising the polymerization temperature. The triad syndiotacticity of the PVA resin can be increased to more than 37.1% by, for example, lowering the polymerization temperature in the example of the production process. In any case, the triad syndiotacticity of the resulting highly syndiotactic PVA can be determined from the peaks attributed to hydroxy groups in the proton NMR analysis of the highly syndiotactic PVA resin dissolved in deuterated DMSO. The highly syndiotactic PVA resin determined to have a triad syndiotacticity of 32 to 40% can be selected as appropriate and used in the present invention.

The vinyl ester polymer containing the above vinyl ester monomers may further contain an additional unsaturated monomer capable of copolymerizing with the vinyl ester monomers to the extent that the additional unsaturated monomer does not impair the effects of the invention.

The additional unsaturated monomer may be one or more unsaturated monomers selected from carboxyl group-containing unsaturated monomers, such as (meth)acrylic acid, maleic acid, maleic anhydride, fumaric acid, crotonic acid, itaconic acid, and undecylenic acid; unsaturated dibasic acid monoalkyl esters, such as monomethyl maleate and monomethyl itaconate; amide group-containing unsaturated monomers, such as acrylamide, dimethylacrylamide, dimethylaminoethylacrylamide, diethylacrylamide, dimethylaminopropylacrylamide, isopropylacrylamide, N-methylolacrylamide, and N-vinylacetamide; vinyl halides, such as vinyl chloride and vinyl fluoride; glycidyl group-containing unsaturated monomers, such as allyl glycidyl ether and glycidyl methacrylate; lactam group-containing unsaturated monomers including, e.g., N-vinylpyrrolidones such as N-vinyl-2-pyrrolidone and N-vinyl-alkylpyrrolidone (e.g., N-vinyl-mono- or di-C₁₋₄ alkyl-pyrrolidones, such as N-vinyl-3-propyl-2-pyrrolidone, N-vinyl-5-methyl-2-pyrrolidone, N-vinyl-5-ethyl-2-pyrrolidone, N-vinyl-5,5-dimethyl-2-pyrrolidone, and N-vinyl-3,5-dimethyl-2-pyrrolidone), N-allylpyrrolidones such as N-allyl-2-pyrrolidone, N-vinylpiperidones such as N-vinyl-2-piperidone and N-vinyl-alkylpiperidone (e.g., N-vinyl-mono- or di-C₁₋₄ alkyl-piperidones, such as N-vinyl-6-methyl-2-piperidone and N-vinyl-6-ethyl-2-piperidone), and N-vinylcaprolactams such as N-vinyl-ε-caprolactam and N-vinyl-alkylcaprolactam (e.g., N-vinyl-mono- or di-C₁₋₄ alkyl-caprolactams, such as N-vinyl-7-methyl-2-caprolactam and N-vinyl-7-ethyl-2-caprolactam); alkyl vinyl ethers such as C₁₋₂₀ alkyl vinyl ethers (e.g., methyl vinyl ether, n-propyl vinyl ether, i-propyl vinyl ether, n-butyl vinyl ether, i-butyl vinyl ether, t-butyl vinyl ether, lauryl vinyl ether, dodecyl vinyl ether, and stearyl vinyl ether); nitriles such as acrylonitrile and methacrylonitrile; hydroxy group-containing unsaturated monomers, including, e.g., C₁₋₂₀ monoalkyl allyl alcohols such as allyl alcohol and isopropenyl allyl alcohol, C₁₋₂₀ dialkyl allyl alcohols such as dimethyl allyl alcohol, and hydroxy C₁₋₂₀ alkyl vinyl ethers such as hydroxy ethyl vinyl ether and hydroxy butyl vinyl ether; acetyl group-containing unsaturated monomers, including, e.g., C₁₋₂₀ alkyl allyl acetates such as allyl acetate, dimethylallyl acetate, and isopropenylallyl acetate; (meth)acrylic acid esters, including, e.g., (meth)acrylic acid alkyl esters, such as (meth)acrylic acid C₁₋₂₀ alkyl esters such as methyl (meth)acrylate, ethyl (meth)acrylate, 2-ethylhexyl acrylate, and n-butyl acrylate; vinylsilanes such as trimethoxyvinylsilane, tributylvinylsilane, and diphenylmethylvinylsilane; polyoxyalkylene (meth)acrylates, such as polyoxyethylene (meth)acrylate and polyoxypropylene (meth)acrylate; polyoxyalkylene (meth)acrylamides, such as polyoxyethylene (meth)acrylamide and polyoxypropylene (meth)acrylamide; polyoxyalkylene vinyl ethers, such as polyoxyethylene vinyl ether and polyoxypropylene vinyl ether; polyoxyalkylene alkylvinyl ethers, such as polyoxyethylene allyl ether, polyoxypropylene allyl ether, polyoxyethylene butylvinyl ether, and polyoxypropylene butylvinyl ether; α-olefins such as ethylene, propylene, n-butene, and 1-hexene; butenes such as 3,4-dihydroxy-1-butene, 3,4-diacyloxy-1-butene, 3-acyloxy-4-hydroxy-1-butene, 4-acyloxy-3-hydroxy-1-butene, and 3,4-diacyloxy-2-methyl-1-butene; pentenes such as 4,5-dihydroxy-1-pentene, 4,5-diacyloxy-1-pentene, 4,5-dihydroxy-3-methyl-1-pentene, and 4,5-diacyloxy-3-methyl-1-pentene; hexenes such as 5,6-dihydroxy-1-hexene and 5,6-diacyloxy-1-hexene; unsaturated amine monomers, such as N,N-dimethylallylamine, N-allylpiperazine, 3-piperidine acrylic acid ethyl ester, 2-vinylpyridine, 4-vinylpyridine, 2-methyl-6-vinylpyridine, 5-ethyl-2-vinylpyridine, 5-butenylpyridine, 4-pentenylpyridine, and 2-(4-pyridyl)allyl alcohol; quaternary ammonium compound-containing unsaturated monomers, such as dimethylaminoethyl acrylate methyl chloride quaternary salt, N,N-dimethylaminopropyl acrylamide methyl chloride quaternary salt, and N,N-dimethylaminopropyl acrylamide methyl benzenesulfonate quaternary salt; aromatic unsaturated monomers such as styrene; sulfonic acid group-containing unsaturated monomers, such as 2-acrylamide-2-methylpropanesulfonic acid or its alkali metal salts, ammonium salts or organic amine salts, 2-acrylamide-1-methylpropanesulfonic acid or its alkali metal salts, ammonium salts or organic amine salts, 2-methacrylamide-2-methylpropanesulfonic acid or its alkali metal salts, ammonium salts or organic amine salts, vinyl sulfonic acid or its alkali metal salts, ammonium salts or organic amine salts, allyl sulfonic acid or its alkali metal salts, ammonium salts or organic amine salts, and methallyl sulfonic acid or its alkali metal salts, ammonium salts or organic amine salts; glycerol monoallyl ether; 2,3-diacetoxy-1-allyloxypropane; 2-acetoxy-1-allyloxy-3-hydroxypropane; 3-acetoxy-1-allyloxy-3-hydroxypropane; 3-acetoxy-1-allyloxy-2-hydroxypropane; glycerol monovinyl ether; glycerol monoisopropenyl ether; acryloyl morpholine; vinyl ethylene carbonate; vinylimidazole; and vinylcarbazole.

The amount of the additional unsaturated monomer may be, for example, but is not limited to, 10 mol or less relative to 100 mol of the vinyl ester monomers.

A polymerization catalyst may be used for the polymerization. The polymerization catalyst may typically be, but is not limited to, an azo compound or a peroxide.

An organic acid, such as tartaric acid, citric acid, and acetic acid, may be added to the polymerization reaction to prevent the hydrolysis of the fatty acid vinyl ester.

A polymerization terminator may be used to terminate the polymerization. The polymerization terminator may be, for example, but is not limited to, m-dinitrobenzene etc.

The polymerization temperature may be set at any temperature known to be used in polymerization. For ease of production of a PVA resin having a high syndiotacticity and other reasons, the polymerization temperature is, for example, -50 to 200°C, preferably - 50 to 150°C, and more preferably 0 to 120°C.

The vinyl ester polymer is produced in the manner as described above. The resulting polymer may be saponified by any conventionally known method. For example, a conventionally known alcoholysis or hydrolysis is applicable using a basic catalyst, such as sodium hydroxide, potassium hydroxide or sodium methoxide, or an acidic catalyst, such as hydrochloric acid, sulfuric acid or p-toluenesulfonic acid. The syndiotacticity of the polymer is usually not greatly changed before and after the saponification reaction.

The solvent used in the saponification may be, for example, an alcohol such as methanol or ethanol; an ester such as methyl acetate or ethyl acetate; a ketone such as acetone or methyl ethyl ketone; an aromatic hydrocarbon such as benzene or toluene; tetrahydrofuran; etc. These solvents may be used alone or in combination of two or more. The temperature, duration, and other conditions of the saponification are not particularly limited.

The drying, grinding or washing of the saponified product may be done by any methods and may be performed by a conventionally known method.

A saponified product of the vinyl ester polymer, i.e., the highly syndiotactic PVA resin according to the present invention is produced in the manner as described above. The produced highly syndiotactic PVA resin may be post-modified by acetalization, urethanation, etherification, grafting, phosphorylation, acetoacetylation, cationization, or other reactions following a known method to the extent that the effects of the present invention are not impaired.

The degree of saponification of the highly syndiotactic PVA resin is preferably 90 to 99.9 mol%, more preferably 98 to 99.9 mol%, and further preferably 99 to 99.9 mol%. The degree of saponification of the highly syndiotactic PVA resin can be determined by, for example, proton NMR analysis in a deuterated DMSO solution.

The degree of polymerization of the highly syndiotactic PVA resin is preferably 100 to 10,000, more preferably 500 to 8,000, and further preferably 1,000 to 5,000. For ease of handling, the degree of polymerization is particular preferably 1,000 to 3,000. The highly syndiotactic PVA resin having a degree of polymerization of 100 or more can be used to easily produce an aqueous gel having a high resin strength (stress) and shape retainability. When the degree of polymerization is 10,000 or less, an aqueous solution of the resin can easily be handled. The degree of polymerization is measured before saponification and is determined in terms of the degree of polymerization of polyvinyl acetate in a benzene solution at 30°C in accordance with JIS K 6725.

### Polyvinyl alcohol resin having degree of saponification of 97 mol% or more

The polyvinyl alcohol resin may be a polyvinyl alcohol resin having a degree of saponification of 97 mol% or more (simply also called the completely saponified PVA resin herein) as described above.

The degree of saponification of the completely saponified PVA resin is preferably 97 mol% or more, e.g., 97 to 99.9 mol%, more preferably 98 mol% or more, e.g., 98 to 99.9 mol%, and particularly preferably 98.5 mol% or more, e.g., 98.5 to 99.9 mol%.

The degree of polymerization of the completely saponified PVA resin is, for example, 100 to 10,000, more preferably 500 to 9,000, further preferably 1,000 to 8,000, and particularly preferably 1,500 to 5,000.

### Crosslinking agent

The cell-embedding device or the polymer that constitutes the cell-embedding device may further comprise a crosslinking agent.

The crosslinking agent may be any crosslinking agent and may be selected depending on the type of polymer or other factors. For example, when the modified PVA resin is used as the polymer to produce the device, a suitable crosslinking agent may be a crosslinking agent having a functional group (e.g., a hydrazino group etc.) capable of reacting with the carbonyl groups of the modified PVA resin.

Examples of the crosslinking agent include hydrazide compounds, semicarbazide compounds, or the like. Particularly preferred are hydrazide compounds, semicarbazide compounds, or the like having two or more functional groups selected from the groups represented by the formulas (1) to (3) below in the molecule. These compounds can be used alone or in combination of two or more.

-NH-NH₂ (1)

-CO-NH-NH₂ (2)

-NH-CO-NH-NH₂ (3)

Specific examples of the hydrazide compounds include carbohydrazide; dicarboxylic acid hydrazides, including aliphatic dicarboxylic acid hydrazides, such as oxalic acid dihydrazide, malonic acid dihydrazide, succinic acid dihydrazide, glutaric acid dihydrazide, adipic acid dihydrazide, pimelic acid dihydrazide, suberic acid dihydrazide, azelaic acid dihydrazide, sebacic acid dihydrazide, dodecanedioic acid dihydrazide, and hexadecanedioic acid dihydrazide; aromatic dicarboxylic acid hydrazides, such as terephthalic acid dihydrazide, isophthalic acid dihydrazide, 2,6-naphthoic acid dihydrazide, and 4,4'-bisbenzene dihydrazide; alicyclic dicarboxylic acid hydrazides, such as 1,4-cyclohexanedicarboxylic acid dihydrazide; hydroxyl group-containing dicarboxylic dihydrazides, such as tartaric acid dihydrazide and malic acid dihydrazide; iminodiacetic acid dihydrazide; itaconic acid dihydrazide; 1,3-bis(hydrazinocarbonoethyl)-5-isopropylhydantoin; 7,11-octadecadiene-1,18-dicarbohydrazide; tris(2-hydrazinocarbonylethyl)isocyanurate; citric acid trihydrazide; butanetricarbohydrazide; 1,2,3-benzenetrihydrazide; ethylenediaminetetraacetic acid tetrahydrazide; 1,4,5,8-naphthoic acid tetrahydrazide; nitriloacetic acid trihydrazide; cyclohexanetricarboxylic acid trihydrazide; pyromellitic acid tetrahydrazide; etc.

Examples of the semicarbazide compounds include N,N'-hexamethylene bissemicarbazide, and biuretry-tri(hexamethylenesemicarbazide).

Derivatives produced by a reaction of these hydrazide compounds or semicarbazide compounds with low boiling point ketones, such as acetone and methyl ethyl ketone, may also be used.

Dicarboxylic acid hydrazides, polyacrylic acid hydrazide and the like are preferred, adipic acid dihydrazide, polyacrylic acid hydrazide and the like are more preferred, and polyacrylic acid hydrazide is particularly preferred among the crosslinking agents as exemplified above. These crosslinking agents are suitable for the invention due to low toxicity, high solubility in water, and other advantages.

The crosslinking agents as described above may be used alone or in combination of two or more.

The amount of the crosslinking agent(s) contained in the polymer is preferably 1 to 30 parts by mass, more preferably 2 to 25 parts by mass, and further preferably 3 to 20 parts by mass, e.g., 4 to 15 parts by mass, relative to 100 parts by mass of the polymer (e.g., the modified PVA resin).

The amount of the crosslinking agent(s) is 1 part by mass or more such that high crosslinking density and sufficient strength (stress) as the cell-embedding device are achieved. The amount of the crosslinking agent(s) is 30 parts by mass or less such that residual unreacted crosslinking agent(s) can be minimized.

When the crosslinking agent is polyacrylic acid hydrazide, the weight average molecular weight (Mw) of the polyacrylic acid hydrazide is preferably, but not limited to, about 3,000 to 6,000,000, more preferably about 5,000 to 1,000,000, and further preferably about 8,000 to 800,000, e.g., about 10,000 to 300,000, about 1,000 to 200,000, or about 10,000 to 100,000.

When the crosslinking agent is polyacrylic acid hydrazide, the degree of hydrazidation of the polyacrylic acid hydrazide is preferably, but not limited to, 30% or more, more preferably 50% or more, further preferably 70% or more, and particularly preferably 80% or more.

The molecular weight and the degree of hydrazidation of polyacrylic acid hydrazide may be adjusted as appropriate to the extent that the effects of the present invention are not impaired. For example, when the molecular weight of polyacrylic acid hydrazide is small, the degree of hydrazidation is adjusted to be large. When the molecular weight of polyacrylic acid hydrazide is large, the degree of hydrazidation is adjusted to be small.

The polyvinyl alcohol resin contained in the cell-embedding device may be in the form of a gel, in particular, an aqueous gel (hydrogel), or a sponge.

The formation of the gel may be performed as appropriate for the type of polymer. For example, the gel may be formed by crosslinking the polymer with a crosslinking agent described later or other agents (gel crosslinking), or a polymer capable of forming a gel (e.g., the highly syndiotactic PVA resin as described above) may be used to form a gel.

The concentration of the polymer in the gel may be, for example, 0.3 to 20%, and is preferably 0.5 to 10%, more preferably 1 to 8%, for example, 3 to 8%. The gel containing such an amount of the polymer is preferred because the gel is capable of maintaining the shape of the encapsulating membrane-forming device implanted in the body of an animal for a long period of time and has other advantages.

When a crosslinking agent is used to form a gel, the amount of the crosslinking agent can be selected as appropriate for the type of crosslinking agent, the desired strength of the gel or other factors. The amount of the crosslinking agent may be, for example, 0.5 parts by mass or more, preferably 1 part by mass or more, more preferably 3 parts by mass or more, etc. relative to 100 parts by mass of the polymer. The amount of the crosslinking agent may be, for example, 20 parts by mass or less, preferably 18 parts by mass or less, more preferably 15 parts by mass or less, etc. relative to 100 parts by mass of the polymer.

When the polyvinyl alcohol resin is formed into a gel, the gel may be shaped or formed into a desired shape by, for example, a method involving pouring a liquid mixture (in particular, an aqueous solution that may be in a sol state) containing a polymer, e.g., the polyvinyl alcohol resin and optionally a crosslinking agent as needed, into a mold of the desired shape to form a gel; or a method involving cutting a produced gel with a knife etc. into the desired shape.

A liquid mixture (in particular, an aqueous solution) containing a polymer, e.g., the polyvinyl alcohol resin and optionally a crosslinking agent as needed typically undergoes a sol state to become a gel. Such a sol is understood to be an equivalent of the gel according to the present invention and is within the scope of the present invention.

The solid content of the liquid mixture (in particular, an aqueous solution that may be in a sol state) is, for example, 0.3 to 20% by mass, preferably 0.5 to 10% by mass, and more preferably 1 to 8% by mass. A gel prepared from the liquid mixture with such a solid content is preferred because the gel is capable of maintaining the shape of the device implanted in the body for a long period of time and retaining an adhesion preventive ability and has other advantages.

The aqueous solution containing the polyvinyl alcohol resin may be prepared in any manner, and may be prepared by a conventionally known method for dissolving a PVA, involving, e.g., dispersing the polyvinyl alcohol resin in water at room temperature, agitating the dispersion while raising the temperature to 80°C or more to allow complete dissolution, and cooling the solution.

The crosslinking agent may be in the form of an aqueous solution. The aqueous solution of the crosslinking agent may be prepared in any manner, and may be prepared by, e.g., a method involving dispersing the crosslinking agent in water at room temperature, and agitating the dispersion at room temperature to allow dissolution. Alternatively, the aqueous solution of the crosslinking agent may be prepared by a method involving dispersing the crosslinking agent in water at room temperature, agitating the dispersion under heating (e.g., at 60°C for 10 minutes) to allow dissolution, and leaving the solution to stand at room temperature.

The aqueous solution of the polyvinyl alcohol resin and the aqueous solution of the crosslinking agent are desirably sterilized by a conventionally known method, such as autoclaving, UV, γ-rays or filtering. The addition of the biological composition under mixing and the subsequent production process of the cell-embedding device are desirably performed under germ-free conditions. The mixture and the cell-embedding device are desirably stored under germ-free conditions.

When the polyvinyl alcohol resin is formed into a sponge, a suitable method for forming a sponge may be, for example, a method involving adding a pore-forming agent in the production process of the gel, or a method involving drying, e.g., lyophilizing, a produced gel to form pores.

The pore-forming agent may be a water-soluble polymer, a water-soluble inorganic substance, an organic solvent, etc. A suitable pore-forming agent is starch.

When starch is used as a pore-forming agent, a crosslinking agent (e.g., formalin) is added to an aqueous solution containing the polyvinyl alcohol resin and starch, then the polyvinyl alcohol resin is gelled, and the starch is washed away with water to give a sponge.

When a pore-forming agent is used to form a sponge, the resulting sponge may be dried.

The cell-containing device may further contain a cell culture component.

The cell culture component may be, for example, but is not limited to, an alkali metal, an alkaline earth metal, a halogen, glucose, etc. Suitable cell culture components are an acetate buffer solution or a phosphate buffer solution containing Na, K, Cl, Ca, glucose and others.

When the cell culture component contains Na, the concentration of Na may be adjusted to preferably 20 to 150 mEq/L, more preferably 80 to 140 mEq/L.

When the cell culture component contains K, the concentration of K may be adjusted to preferably 2.5 to 130 mEq/L, more preferably 3.5 to 40 mEq/L.

When the cell culture component contains Cl, the concentration of Cl may be adjusted to preferably 15 to 170 mEq/L, more preferably 100 to 150 mEq/L.

When the cell culture component contains Ca, the concentration of Ca may be adjusted to preferably 0.5 to 5 mEq/L, more preferably 1 to 3 mEq/L.

When the cell culture component contains glucose, the concentration of glucose may be adjusted to preferably 1 to 11 mM, more preferably 3 to 7 mM.

The cell culture component may also be, for example, a known cell culture medium, such as Hanks' balanced salt solution (HBSS); a commercially available preservation solution, such as Euro-Collins solution, CELLBANKER, and UW solution (University of Wisconsin solution); a cell protective component, such as dimethylsulfoxide (DMSO) and serum albumin; a component for preventing the contamination by germs, such as antibiotics; a component for maintaining the activity of cells, such as vitamins such as nicotinamide; etc. The cell culture component is preferably a known cell culture medium etc.

These cell culture components can be used alone or in combination of two or more.

Such a cell culture component may be used in combination with another component, such as a sustained release agent, an isotonic agent, and a pH adjusting agent.

The cell-containing device may further contain a component other than those described above. The cell-containing device may further contain, for example, a growth factor (a cell growth factor), a cytokine, other physiologically active substances, a blood-flow accelerator, a neurotrophic factor, etc.

These components can be used alone or in combination of two or more.

Examples of the growth factor (cell growth factor) include the growth factors etc. as exemplified above, such as epidermal growth factor (EGF), hepatocyte growth factor (HGF) and insulin.

Examples of the cytokine include hematopoietic factors, such as interleukins, chemokines and colony-stimulating factors, tumor necrosis factors, and interferons.

Examples of other physiologically active substances include amino acids, such as glycine, phenylalanine, lysine, aspartic acid, and glutamic acid; vitamins, such as biotin, pantothenic acid, and vitamin D; serum albumin; and antibiotics.

Examples of the blood-flow accelerator include citrulline or salts thereof, capsaicin, and capsaicinoids.

Examples of the neurotrophic factor include nerve growth factor (NGF), brain-derived neurotrophic factor (BDNF), neurotrophin-3 (NT-3), neurotrophin-4 (NT-4), glial cell-derived neurotrophic factor (GDNF), neurturin, artemin, and persephin.

The amount of these components added to the cell-containing device is not limited to a particular amount.

The cell-containing device or the cell-embedding device may form a gel.

The gel typically has a predetermined strength (stress). The gel may sustain a sufficient stress not to easily break when the device is implanted. The strength of the gel may vary depending on the type of polymer (e.g., the type of polyvinyl alcohol resin, the viscosity of a 4% aqueous solution of the resin, the degree of saponification, the degree of modification, syndiotacticity, etc.), the type of crosslinking agent, the amount of the crosslinking agent added, the solid content of the gel, etc. The strength of the gel is therefore not limited to a specific value, but the gel may be able to sustain a stress of, for example, 0.5 to 100 kPa, preferably 0.6 to 95 kPa, more preferably 0.7 to 90 kPa, and further preferably 0.7 to 85 kPa, as measured at 20°C.

The stress sustained by the gel can be measured using, for example, a compact tabletop-tester EZ Test EZ-SX (Shimadzu Corporation) following the manufacturer's instructions.

The gel, in particular the aqueous gel, may be in any shape, and may be in the shape of, for example, not limited to, a sheet, a plate, a disk, a bar, a tube, a bead, etc.

The size of the gel, in particular the aqueous gel, may be selected as appropriate for the implantation site of the device and its size, etc., and is not particularly limited. For example, the thickness of the gel is preferably 0.1 to 10 mm, more preferably 0.2 to 5 mm, further preferably 0.5 to 2 mm, and particularly preferably 0.7 to 1.5 mm.

The cell-containing device may further contain a supporting substrate.

In particular, the gel, for example, the gel that forms a scaffold, may contain a supporting substrate useful as a reinforcement for the purpose of reinforcing the gel and/or for ease of handling.

For example, when the gel (in particular, in the form of an aqueous gel) is formed into a thin film, a polymer is placed on a substrate (a reinforcement), such as a mesh sheet made of a resin, for reinforcement and/or for ease of handling, and is then formed into a gel.

The material of the supporting substrate may be, but is not limited to, a polymer such as PET (polyethylene terephthalate), PE (polyethylene), PP (polypropylene), and Teflon (registered trademark); a metal; etc. The material of the supporting substrate preferably does not degrade or deteriorate in a living body, but may be a biodegradable material that degrades in a living body after a certain period of time has passed.

The mesh size of the mesh sheet is such a size that allows permeation of molecules that should be permeated through the mesh sheet and have an assumed maximum diameter of about 5 nm, such as oxygen, inorganic or organic nutrients and various hormones (e.g., physiologically active substances including hormones, such as insulin) while preventing permeation of molecules that should be prevented from permeating through the mesh sheet and have an assumed minimum diameter of about 50 nm, such as immune-related cells and immune-related substances (e.g., antibodies, complements, etc.). The mesh size of the mesh sheet is typically 5 to 100 nm, preferably 10 to 50 nm, and more preferably 20 to 30 nm.

The cell-containing device or the cell-embedding device may have, for example, an immunoisolation layer containing a polymer, e.g., the polyvinyl alcohol resin (A). In other words, the cell-containing device may have an immunoisolation layer made of a polymer (a polymer in a gel form) or may have an immunoisolation function exhibited by a polymer (a polymer in a gel form).

The immunoisolation layer or the immunoisolation function refers to a layer or function that prevents permeation of, for example, immune-related proteins, such as antibodies, complements and leucocytes, while allowing permeation of, for example, glucose; hormones such as insulin, thyroid-stimulating hormone, thyroid hormones, parathyroid hormone, growth hormone, thyroxine, glucocorticoids, glucagon, estradiol, and testosterone; proteins such as blood coagulation factors, albumin, globulin, and various enzymes (metabolic enzymes or digestive enzymes such as amylase, protease, and lipase); neurotransmitters, such as dopamine; etc.

The cell-containing device or the cell-embedding device may be, for example, a bioartificial organ etc.

The cell-containing device or the cell-embedding device can be produced by, for example, combining a biological composition and a polymer, and optionally an additional component.

The cell-containing device or the cell-embedding device can be produced by a conventional method selected as appropriate for the types, forms, configurations, etc. of the components.

For example, a liquid mixture containing a polymer and optionally an additional component is preferably formed into a gel.

When the polymer is formed into a gel, the gel may be shaped or formed into a desired shape by, for example, a method involving pouring a liquid mixture (in particular, an aqueous solution that may be in a sol state) containing a polymer, e.g., a polyvinyl alcohol resin and optionally a biological composition, a crosslinking agent and a cell culture component as needed, into a mold of the desired shape to form a gel; or a method involving cutting a produced gel with a knife etc. into the desired shape.

A liquid mixture (in particular, an aqueous solution) containing a polymer, e.g., the polyvinyl alcohol resin and optionally a biological composition, a crosslinking agent and a cell culture component as needed typically undergoes a sol state to become a gel. Such a sol is understood to be an equivalent of the gel according to the present invention and is within the scope of the present invention.

The solid content of the liquid mixture (in particular, an aqueous solution that may be in a sol state) is, for example, 0.3 to 20% by mass, preferably 0.5 to 10% by mass, and more preferably 1 to 8% by mass. A gel prepared from the liquid mixture with such a solid content is preferred because the gel is capable of maintaining the shape of the cell-embedding device implanted in the body for a long period of time and retaining an adhesion preventive ability and has other advantages.

The liquid mixture may be prepared by mixing all the components together. Alternatively, a liquid mixture containing a polymer and optionally a crosslinking agent and a cell culture component as needed (e.g., an aqueous solution of a polyvinyl alcohol resin) may be first prepared, and a biological composition may be added to the mixture.

Other components may be added together with or separately from the biological composition and/or the cell culture component.

The aqueous solution containing a polyvinyl alcohol resin may be prepared in any manner, and may be prepared by a conventionally known method for dissolving a PVA, involving, e.g., dispersing a polyvinyl alcohol resin in water at room temperature, agitating the dispersion while raising the temperature to 80°C or more to allow complete dissolution, and cooling the solution.

The crosslinking agent may be in the form of an aqueous solution. The aqueous solution of the crosslinking agent may be prepared in any manner, and may be prepared by, e.g., a method involving dispersing the crosslinking agent in water at room temperature, and agitating the dispersion at room temperature to allow dissolution. Alternatively, the aqueous solution of the crosslinking agent may be prepared by a method involving dispersing the crosslinking agent in water at room temperature, agitating the dispersion under heating (e.g., at 60°C for 10 minutes) to allow dissolution, and leaving the solution to stand at room temperature.

The aqueous solution of the polyvinyl alcohol resin and the aqueous solution of the crosslinking agent are desirably sterilized by a conventionally known method, such as autoclaving, UV, γ-rays or filtering. The addition of a biological composition under mixing and the subsequent production process of the cell-containing device are desirably performed under germ-free conditions. The mixture and the cell-containing device are desirably stored under germ-free conditions.

The aqueous solution of the polyvinyl alcohol resin and the mixture or aqueous solution containing the polyvinyl alcohol resin and the crosslinking agent and optionally a biological composition and/or a cell culture component as needed used in the preparation of the cell-embedding device may be left to stand for a certain period of time.

The temperature at which the mixture or the aqueous solution is allowed to stand may be a temperature suitable for storage of the biological composition.

The duration during which the mixture or the aqueous solution is allowed to stand to prepare a gel (time for gelation or gelation time) may be selected as appropriate for the concentration of the polymer (the polyvinyl alcohol resin etc.), the amount of the crosslinking agent, the temperature at which the mixture or the aqueous solution is allowed to stand, or other factors, but is usually about 1 hour to about one week at normal temperature. The duration during which the mixture or the aqueous solution is allowed to stand is preferably one hour or more so that the cell-embedding device does not easily break when placed in the body.

When a modified PVA resin is used for the preparation of the cell-embedding device, a pH buffer solution or the like may be added to a liquid mixture containing a modified PVA resin and a crosslinking agent and optionally MSCs and/or a cell culture component as needed to adjust the pH of the liquid mixture and control the gelation time. When the pH of the system is lowered, the gelation time tends to be shortened. When the pH of the system is raised, the gelation time tends to be extended.

An example of a production process of the cell-containing or cell-embedding device using a supporting substrate will be described below.

A liquid polymer mixture (e.g., an aqueous solution or an aqueous gel containing a polyvinyl alcohol resin) containing a cell culture component, optionally in a gel form, is placed on a base material or a base plate (e.g., a glass slide). A supporting substrate, such as a PET mesh (e.g., trade name: PET mesh sheet (TN120), Sanplatec Corp.) is stacked on the polymer mixture. A mixture or solution or dispersion or suspension containing a biological composition dissolved or dispersed or suspended in a liquid polymer mixture (e.g., an aqueous solution or an aqueous gel containing a polyvinyl alcohol resin), optionally in a gel form, is spread over the supporting substrate using a gel loading tip etc. Another supporting substrate (e.g., a PET mesh) is stacked on the polymer mixture-loaded PET mesh in such a manner that the polymer mixture is sandwiched between the supporting substrates. A liquid polymer mixture (e.g., an aqueous solution or an aqueous gel containing a polyvinyl alcohol resin) containing a cell culture component, optionally in a gel form, is placed on the supporting substrate (e.g., a PET mesh). Another base material or base plate (e.g., a glass slide) is stacked on the supporting substrate. The base materials or the base plates (e.g., glass slides) are removed from the assembled polymer mixture and the supporting substrates to give a cell-containing device according to an aspect of the present invention.

The present invention also includes an implantation method, in particular, a method for implanting the cell-containing device, as described above. In the implantation method, the cell-containing device is implanted into the site from which the device 1 has been implanted and subsequently at least the non-bioabsorbable material has been retrieved as described above. According to such an implanting method, various diseases or symptoms can be prevented or improved depending on the biological composition (cells or tissue) used. The present invention thus also includes a method for preventing and/or treating or improving a disease or a symptom.

Such a method includes implanting the cell-containing device after the device 1 has been implanted and subsequently at least the non-bioabsorbable material has been retrieved, and may, of course, include implanting or placing the device 1 before the implantation of the cell-containing device.

Examples of the disease or symptom include, for example, endocrine diseases, such as thyroid diseases, parathyroid diseases, adrenal diseases, pituitary diseases, and pineal diseases; metabolic diseases, such as ornithine transcarbamylase deficiency, hyperammonemia, hypercholesterolemia, homocystinuria, glycogenosis, Crigler-Najjar syndrome, and Wilson's disease; diabetes mellitus, such as type 1 diabetes mellitus, type 2 diabetes mellitus, and pancreatic diabetes; neurodegenerative diseases, such as Parkinson's disease, Alzheimer's disease, amyotrophic lateral sclerosis, and spinocerebellar degeneration; hemophilia; bone diseases such as osteoporosis; cancers such as leukemia; etc.

The duration of the implantation or placement of the cell-containing device may be, for example, but is not limited to, 10 days or more, one month or more, two months or more, three months or more, six months or more, one year or more, etc.

The device 1 of the present invention is suitable for used in combination with the cell-containing device as described above. The present invention thus includes a combination device (or a kit, a device or a combination) comprising the device 1 and the cell-containing device.

### EXAMPLES

The present invention will be described in more detail with reference to Examples, but is not limited thereto. Various modifications may be made by a person having ordinary skill in the art, without departing from the technical idea of the present invention.

### Density

The density of a sample was determined by measuring the volume and the mass.

The density of a material (A) serving as a bioabsorbable material (a gelatin) of the device was determined as follows. First, the moisture percentage of the bioabsorbable material or the bioabsorbable member was determined using a thermogravimetry/differential thermal analyzer (TG-DTA). The weight of the bioabsorbable material or the bioabsorbable member was corrected with the moisture percentage. The volume of the bioabsorbable material was separately measured. The density of the bioabsorbable material was then determined from the corrected weight and the volume of the bioabsorbable material.

### Dissolution rate

A material (A) was immersed in physiological saline at 37°C so that the amount of the material (A) in the physiological saline was 5% by mass. After 24 hours of immersion, the mass of the solid content of the physiological saline was measured and used for the calculation of the dissolution rate.

The dissolution rate of the material (A) serving as a bioabsorbable material (a gelatin) of the device was corrected with the moisture percentage of the material (A), as with the measurement of the density of the material (A).

### Degree of swelling

The degree of swelling of a material (A) was determined after immersion of the material (A) in physiological saline at 37°C for 60 minutes, and calculated based on the mass of the material (A) before and after the immersion.

The degree of swelling of the material (A) serving as a bioabsorbable material (a gelatin) of the device was corrected with the moisture percentage of the material (A), as with the measurement of the density of the material (A).

### Analysis of modified PVA resins

The viscosity of a 4% aqueous solution of a modified PVA resin and the degree of saponification of a modified PVA resin were determined in accordance with JIS K 6726

(1994). The amount of diacetone acrylamide units, i.e., the degree of modification was determined from the integral value of the peaks attributed to the units in ¹H-NMR analysis using DMSO-d₆ as the solvent.

### Analysis of crosslinking agent (polyacrylic acid hydrazide)

The weight average molecular weight of a crosslinking agent was determined by size exclusion chromatography under the following conditions.

### Measurement conditions:

Solvent: a 50 mM aqueous solution of sodium dihydrogen phosphate
Polymer concentration: 1 mg/mL
Flow rate: 1.0 mL/min
Column temperature: 40°C
Column: Shodex OHPak SB-803HQ, Shodex OHPak SB-805HQ
Standard: pullulan
Detector: RI

The degree of hydrazidation was determined by back titration of I₂ using a sodium thiosulfate standard solution. The details of the experimental procedures are as follows.

### Experimental procedures:

1. An I₂/MeOH solution was prepared.
2. The I₂/MeOH solution was titrated using a 0.1 M sodium thiosulfate standard solution (the concentration was determined to be 0.047 M in this measurement).
3. A polymer sample was precisely weighed and dissolved in 20 mL of ion exchanged water.
4. The 0.047 M I₂/MeOH solution was added to 2.0 mL of the solution prepared in step 3.
5. Back titration of I₂ was performed using the 0.1 M sodium thiosulfate standard solution.

### Evaluation of device

The device of the invention (the implantation device or the device 1) was implanted or placed into a subcutaneous site (adipose tissue) in rats and formation of an encapsulating membrane was induced. The device was retrieved, and a pancreatic islet-containing device was then implanted into the subcutaneous site. The device of the invention was evaluated through the evaluation of the functions of the pancreatic islet-containing device.

In particular, a pancreatic islet-containing device was implanted into streptozotocin-induced diabetic rats, and the blood glucose levels were measured over time to evaluate the therapeutic effect of the device on diabetes mellitus.

### Example 1

### Preparation of material (A)

A 5% by mass aqueous solution of a commercially available gelatin (RM-50, acid-treated gelatin from porcine skin, Jellice Co., Ltd.) was lyophilized. A 22-mm diameter cylinder with a thickness of 0.5 mm was punched out from the lyophilized gelatin, and dried under vacuum at 145°C for 24 hours to crosslink the gelatin to give a material (A).

The material (A) had a density of 58 mg/cm³, a dissolution rate of 19.8% and a degree of swelling of 9.1-fold, and the ratio of the density to the degree of swelling was 6.4.

### Preparation of non-bioabsorbable material

A 26-mm diameter cylinder with a thickness of 0.5 mm was punched out from a commercially available silicone rubber (Cat. No. 6-611-02, thickness: 1 mm, AS ONE Corporation) and used as a non-bioabsorbable material (B).

The non-bioabsorbable material (B) had a density of 1200 mg/cm³, a dissolution rate of 0% and a degree of swelling of 1.0-fold, and the ratio of the density to the degree of swelling was 1200.

### Production of device

Two pieces of the material (A) were left to be immersed in physiological saline at 22°C for 1 minute to allow the fabric to sufficiently swell.

Each of the two pieces of the material (A) swollen with saline was mounted on either of the front and back sides of the non-bioabsorbable material (B), and fixed on the non-bioabsorbable material (B) by suturing with a suture to produce a device of the invention (an implantation device or device 1).

### Implantation and placement of device

The device of the invention (the implantation device or the device 1) was subcutaneously placed in 12- to 14-week-old male Wistar rats (Japan SLC) for six weeks. There was no bleeding or release of exudates in the subcutaneous site prior to the placement of the device, and an encapsulating membrane was not observed. After placing the device for six weeks, sufficient formation of an encapsulating membrane in the subcutaneous site was observed even with the naked eye, as compared with before the placement of the device.

The material (A) of the device was nearly completely absorbed by the body and the surrounding tissue and formed an encapsulating membrane, and only the non-bioabsorbable material remained at the placement site (implantation site). There were few adhesions between the surface of the non-bioabsorbable material and the subcutaneous tissue, and the non-bioabsorbable material was easily separated from the subcutaneous tissue. After the non-bioabsorbable material was retrieved, no bleeding or release of exudates at all was observed in the subcutaneous site from which the non-bioabsorbable material was retrieved.

Due to these, after retrieval of the device, implantation of another cell, tissue or device was easily carried out on the site. For example, the pancreatic islet-embedding device as described later was easily inserted into the site.

The implantation or placement of the device of the invention was performed on two rats, and the rats showed similar results, i.e., the rats showed formation of a sufficient amount of an encapsulating membrane and no bleeding or release of exudates when the device of the invention was retrieved.

### Preparation of pancreatic islet cells for pancreatic islet-embedding device

Pancreatic islets were isolated from 11- to 14-week-old male Lewis rats (Japan SLC). Cold Hanks' balanced salt solution (HBSS) containing 0.8 mg/mL collagenase type V (Sigma-Aldrich) was injected through the common bile duct into the pancreas of the rats to digest the pancreas at 37°C for 12 minutes to separate pancreatic islets from the pancreatic tissue. Concentration gradient centrifugation was performed using Histopaque-1119 (Sigma-Aldrich) and Lymphoprep (Axis-Shield, Norway) to collect the pancreatic islets. The pancreatic islets were cultured in RPMI 1640 medium containing 5.5 mmol/L glucose and 10% fetal bovine serum (FBS) under 5% CO₂ at 37°C overnight.

### Synthesis of polymer for pancreatic islet-embedding device

Into a flask equipped with an agitator, a thermometer and a dropping-funnel with a reflux condenser were placed 2,000 parts of vinyl acetate, 143 parts of methanol and 3.7 parts of diacetone acrylamide. The air in the system was replaced with nitrogen, and the internal temperature was raised to 60°C. A solution of 0.16 parts of 2,2-azobis(isobutyronitrile) in 100 parts of methanol was added to initiate polymerization. Immediately after the initiation of polymerization, a solution of 70.1 parts of diacetone acrylamide in 46.7 parts of methanol was added dropwise at a constant rate while allowing nitrogen to flow through the flask. At 210 minutes after the initiation of polymerization, m-dinitrobenzene was added as a polymerization terminator to stop the polymerization. The yield at the end of polymerization was 47.1%. Methanol vapor was added to the resulting reaction mixture to distill off the remaining vinyl acetate to give a 35% solution of a diacetone acrylamide-vinyl acetate copolymer in methanol. To 500 parts of this solution, 70 parts of methanol, 1 part of ion exchanged water and 29.3 parts of a 4% solution of sodium hydroxide in methanol were added, and the mixture was thoroughly agitated at 45°C to allow a saponification reaction to proceed. The resulting gelatinous material was pulverized, washed thoroughly with methanol and dried to give D-PVA 1. A 4% aqueous solution of D-PVA 1 had a viscosity of 53.4 mPa·s. D-PVA 1 had a degree of saponification of 98.4 mol% and contained 3.6 mol% diacetone acrylamide units.

### Synthesis of crosslinking agent for pancreatic islet-embedding device

To an aqueous solution of 20 parts of polyacrylamide with a weight average molecular weight of about 40,000 in 40 parts of ion exchanged water was added 16 parts of hydrazine monohydrate, and the mixture was reacted at 80°C for 15 hours. Ethanol was added to the liquid mixture, and the precipitate was filtered, washed and dried to give APA 1. APA 1 had a weight average molecular weight of about 53,000 and a degree of hydrazidation of 88%.

### Preparation of aqueous solution of modified PVA resin and crosslinking agent in a sol state

To a 25 mL tube were added 8.0 g of a 6.25% aqueous solution of D-PVA 1 prepared in Synthetic Example 1 and 1.0 mL of 10 × HBSS (Hanks' balanced salt solution), and the mixture was agitated by shaking the tube up and down.

The mixture was spin down in a centrifuge (trade name: Hybrid high-speed refrigerated centrifuge 6200, Kubota Corporation), and the mixture was left to stand at 37°C for 10 minutes. Then, 1.0 mL of a 5% aqueous solution of APA 1 prepared in Synthetic Example 9 was added as a crosslinking agent, and the tube was shaken up and down 15 times. The mixture was spin down in the centrifuge, and the tube was again shaken up and down 15 times.

The mixture was centrifuged at 3,000 rpm at 25°C for 1 minute and left to stand at 37°C. The viscosity of the aqueous solution in a sol state was checked at appropriate intervals until the sol beaded up within 3 to 4 seconds, indicating that the sol was in a state optimal for embedding pancreatic islets. The tube was taken out of the hot bath, and left to stand on ice for 1 minute. The tube was centrifuged at 3,000 rpm at 25°C for 1 minute to give a sol containing the modified PVA resin in an amount of 5% and the crosslinking agent in an amount of 0.5%.

The concentration of D-PVA 1 in the gel (hydrogel) was 5.0%, and the concentration of APA 1 in the gel was 0.5%. The stress sustained by the gel prepared in accordance with the formulation was 5.2 kPa.

### Preparation of pancreatic islet-embedding device

An amount of 160 µL of the sol prepared on a dish was placed on a glass slide. A PET mesh (trade name: PET mesh sheet (TN120), Sanplatec Corp.) was placed on the sol. The culture medium components were removed from the prepared pancreatic islet cells as thoroughly as possible, and the cells were suspended in 50 µL of an aliquot of the sol to prepare a suspension. The suspension of the pancreatic islet cells was spread over the PET mesh. Another PET mesh was further stacked on the sol-loaded PET mesh in such a manner that the suspension of the pancreatic islet cells (18,000 Islet Equivalents (IEQ): IEQ is an international unit of the number of pancreatic islets; one IEQ is defined as a single islet of 150 µm in diameter) was sandwiched between the PET meshes. On the PET meshes, 140 µL of the sol was placed. Another glass slide was then mounted on the sol. The assembled sol and PET meshes were placed in a moist chamber, and left to stand at 4°C for 48 hours to prepare a pancreatic islet-embedding device (in the form of an aqueous gel).

### Storing of pancreatic islet-embedding device

The pancreatic islet-embedding device assembled as above was taken off the glass slides, immersed in 5 mL/well of a preservation medium (RPMI 1640 medium containing 10% FBS and glucose adjusted to a concentration of 5.5 mM) in a 6-well plate, and stored at 4°C for about 16 hours.

### Implantation step

The device 1 was placed in rats, and about five weeks after the placement of the device 1 (about one week prior to implantation of the pancreatic islet-embedding device), streptozotocin was injected to induce diabetes mellitus.

At six weeks after the placement of the device 1, the non-bioabsorbable material 2 was retrieved. The pancreatic islet-embedding device (in the form of an aqueous gel) after storage was implanted by placing it into the subcutaneous site in the rats, i.e., the implantation site of the device 1 or the subcutaneous site from which the non-bioabsorbable material 2 was retrieved.

At the time of the implantation of the pancreatic islet-embedding device, an encapsulating membrane, i.e., an encapsulating membrane containing the non-woven fabric 1 or the gelatin, was retained.

### Evaluation of therapeutic effect on diabetes mellitus

After implantation of the pancreatic islet-embedding device, the blood glucose levels were measured over time to evaluate the therapeutic effect.

The evaluation of the therapeutic effect on diabetes mellitus was performed on two rats.

### Example 2

A 30-mm diameter cylinder with a thickness of 0.5 mm was punched out from a commercially available gelatin (Spongel, LTL Pharma Co., Ltd.), and about 400 pores were created using an injection needle. The gelatin was used as a material (A).

The material (A) had a density of 15 mg/cm³, a dissolution rate of 33.4% and a degree of swelling of 48.5-fold, and the ratio of the density to the degree of swelling was 0.303.

The evaluation of the therapeutic effect on diabetes mellitus was performed in the same manner as in Example 1 except that the material (A) prepared above was used in place of the material (A) of Example 1.

The prepared device showed a similar tendency to that of Example 1 during placement until retrieval, i.e., the rats showed formation of a sufficient amount of an encapsulating membrane and no bleeding or release of exudates when the device was retrieved.

### Example 3

The evaluation of the therapeutic effect on diabetes mellitus was performed in the same manner as in Example 1 except that a device not containing a material (A) (a device containing the non-bioabsorbable material (B) only, i.e., a device containing the non-bioabsorbable material as a material (A)) was used in place of the device of Example 1.

The prepared device showed a similar tendency to that of Example 1 during placement until retrieval, i.e., the rats showed no bleeding or release of exudates when the device was retrieved. Formation of an encapsulating membrane was observed, but the thickness of the encapsulating membrane was smaller than that observed in Examples 1 and 2.

### Reference Example 1

The implantation and the evaluation of the therapeutic effect on diabetes mellitus were performed in the same manner as in Example 1 except that a commercially available gelatin sponge (Gelfoam, Pfizer, Inc.) was used in place of the material (A) of Example 1.

### Reference Example 2

A commercially available gelatin (RM-50, Jellice Co., Ltd.) was used in place of the material (A) of Example 1, but the gelatin was dissolved in physiological saline, and the device was not able to be produced.

### Comparative Example 1

The evaluation of the therapeutic effect on diabetes mellitus was performed in the same manner as in Example 1 except that the implantation or placement of the device 1 was not performed.

The results of the evaluation of the therapeutic effect on diabetes mellitus in Examples 1 to 3, Reference Example 1 and Comparative Example 1 are summarized in Table 1 below.

The blood glucose levels before implantation refer to the blood glucose levels measured just before the pancreatic islet device was implanted after the retrieval of the device 1.

**Table 1**

| | Material (A) constituting implantation device | | | | Rat No. | Pancreatic islet device | | |
|---|---|---|---|---|---|---|---|---|
| | Density X (mg/cm³) | Dissolution rate (% by mass) | Degree of swelling Y (fold by mass) | X/Y | | Blood glucose levels before implantation (mg/dL) | Blood glucose levels 30 days after implantation (mg/dL) | Blood glucose levels 60 days after implantation (mg/dL) |
| Example 1 | 58 | 19.8 | 9.1 | 6.4 | 1 | 483 | 99 | 71 |
| | | | | | 2 | 497 | 99 | 62 |
| Example 2 | 15 | 33.4 | 49.5 | 0.303 | 1 | 421 | 111 | 302 |
| | | | | | 2 | 439 | 51 | 128 |
| Example 3 | 1200 | 0 | 1.0 | 1200 | 1 | 435 | 141 | 113 |
| | | | | | 2 | 456 | 69 | 92 |
| Reference Example 1 | 10 | 20.6 | 49.3 | 0.203 | 1 | 452 | 443 | 468 |
| | | | | | 2 | 440 | 439 | 443 |
| Comparative Example 1 | None | | | | 1 | 407 | 485 | 402 |
| | | | | | 2 | 409 | 306 | 375 |
| | | | | | 3 | 497 | 370 | 369 |
| | | | | | 4 | 460 | 315 | 340 |

As apparent from the results shown in the table above, the implantation device of Examples activated the subcutaneous implantation site and allowed the subsequently implanted pancreatic islet device to effectively exhibit its functions. Formation of an encapsulating membrane at the implantation site was also observed for the implantation devices of Examples 1 to 3, and in particular, formation of a sufficient amount of an encapsulating membrane was observed in Examples 1 and 2.

Such an ability of the devices of Examples 1 to 3 to allow the pancreatic islet device to effectively exhibit its functions was unexpected since the devices of Examples 1 to 3 were free of growth factors or other factors. Based on the inventors' investigations, the results indicate increase of an endogenous growth factor, an endogenous extracellular matrix, etc.

The devices of Examples 1 to 3 induced such activation and caused no bleeding or release of exudates in the implantation site when the devices were retrieved. In this manner, the devices of Examples 1 to 3 facilitated efficient implantation of another device and allowed the implanted device to efficiently exhibit or achieve its functions.

### INDUSTRIAL APPLICABILITY

The present invention provides a novel device and others. The device of the invention is applicable as a device for activating an implantation site, for example, a device for restoring an injury, or a device adapted to allow a separately implanted cell-containing device to exhibit its intrinsic, sufficiently high performance.

## Claims

1. An implantation device comprising a material (A) having a density of 12 mg/cm³ or more and a dissolution rate of 80% by mass or less as determined after immersion of the material (A) in physiological saline at 37°C for 24 hours.

2. An implantation device comprising a material (A) having a dissolution rate of 80% or less as determined after immersion of the material (A) in physiological saline at 37°C for 24 hours and a ratio of X/Y of 0.22 or more wherein X is a density (mg/cm³) of the material (A) and Y is a degree of swelling of the material (A) expressed in terms of fold increase calculated on mass basis after immersion of the material (A) in physiological saline at 37°C for 60 minutes.

3. An implantation device comprising a material (A) having a density of 12 mg/cm³ or more, a dissolution rate of 80% by mass or less as determined after immersion of the material (A) in physiological saline at 37°C for 24 hours and a ratio of X/Y of 0.22 or more wherein X is a density (mg/cm³) of the material (A) and Y is a degree of swelling of the material (A) expressed in terms of fold increase calculated on mass basis after immersion of the material (A) in physiological saline at 37°C for 60 minutes.

4. The device according to any one of claims 1 to 3, wherein the material (A) has a dissolution rate of 60% by mass or less as determined after immersion of the material (A) in physiological saline at 37°C for 24 hours.

5. The device according to any one of claims 1 to 4, wherein the material (A) has a degree of swelling of 70-fold or less calculated on mass basis after immersion of the material (A) in physiological saline at 37°C for 60 minutes.

6. The device according to any one of claims 1 to 5, wherein the material (A) has a ratio of X/Y of 0.23 or more wherein X is a density (mg/cm³) of the material (A) and Y is a degree of swelling of the material (A) expressed in terms of fold increase calculated on mass basis after immersion of the material (A) in physiological saline at 37°C for 60 minutes.

7. The device according to any one of claims 1 to 6, wherein the material (A) has a density of 14 mg/cm³ or more, a dissolution rate of 3 to 50% by mass as determined after immersion of the material (A) in physiological saline at 37°C for 24 hours and a ratio of X/Y of 0.23 or more wherein X is a density (mg/cm³) of the material (A) and Y is a degree of swelling of the material (A) expressed in terms of fold increase calculated on mass basis after immersion of the material (A) in physiological saline at 37°C for 60 minutes.

8. The device according to any one of claims 1 to 7, wherein the material (A) contains a bioabsorbable material (A1).

9. The device according to any one of claims 1 to 8, wherein the material (A) contains a gelatin.

10. The device according to any one of claims 1 to 9, wherein the material (A) forms at least part of a surface of the device.

11. The device according to any one of claims 1 to 10, wherein the device comprises a material (A) and a non-bioabsorbable material (B).

12. The device according to any one of claims 1 to 11, wherein the device comprises a material (A) and a non-bioabsorbable material (B), wherein the material (A) contains a bioabsorbable material (A1), and wherein the material (A) is integrated with the non-bioabsorbable material (B).

13. The device according to any one of claims 1 to 12, wherein the device comprises a material (A) and a non-bioabsorbable material (B), wherein the material (A) contains a bioabsorbable material (A1), and wherein the non-bioabsorbable material (B) is contained in an amount of 1 part by volume or more relative to 100 parts by volume of the material (A).

14. The device according to any one of claims 1 to 13, wherein the device comprises a material (A) and a non-bioabsorbable material (B), wherein the material (A) contains a bioabsorbable material (A1), wherein the material (A) is integrated with the non-bioabsorbable material (B), wherein the non-bioabsorbable material (B) is contained in an amount of 10 parts by volume or more relative to 100 parts by volume of the material (A), and wherein the material (A) forms at least part of a surface of the device.

15. The device according to any one of claims 11 to 14, wherein the non-bioabsorbable material (B) has an adhesion preventive ability.

16. The device according to any one of claims 1 to 15, wherein the device is substantially free of growth factors.

17. The device according to any one of claims 1 to 16, wherein the device is for formation of an encapsulating membrane.

18. The device according to any one of claims 1 to 17, wherein the device is for implantation into an implantation site of a cell- or tissue-containing device.

19. The device according to any one of claims 1 to 18, wherein the device is for use in combination with a cell- or tissue-containing device.

20. The device according to claim 18 or 19, wherein the cell- or tissue-containing device has an immunoisolation layer containing a polyvinyl alcohol resin (A).

21. The device according to any one of claims 18 to 20, wherein the cell- or tissue-containing device comprises an immunoisolation layer containing at least one or more polyvinyl alcohol resins (A) selected from a modified polyvinyl alcohol resin having an active carbonyl group (A1), a polyvinyl alcohol resin having a triad syndiotacticity of 32 to 40% (A2), and a polyvinyl alcohol resin having a degree of saponification of 97 mol% or more (A3).

22. The device according to any one of claims 18 to 22, wherein the cell or tissue contains at least one selected from a pancreatic islet, hepatocytes, stem cells that give rise to islet cells or hepatocytes, and progenitor cells that give rise to islet cells or hepatocytes.

23. A method for implanting the device according to any one of claims 1 to 22.

24. A method for activating an implantation site, comprising implanting the device according to any one of claims 1 to 22 into the implantation site.

25. The method according to claim 23 or 24, wherein a material containing a bioabsorbable material (A1) is used as a material (A), and wherein the bioabsorbable material (A1) is biologically absorbed.

26. The method according to any one of claims 23 to 25, wherein a material containing a bioabsorbable material (A1) is used as a material (A) to allow formation of an encapsulating membrane at the implantation site.

27. A method comprising implanting the device according to any one of claims 1 to 22, wherein the device comprises a non-bioabsorbable member as a material (A) or comprises a non-bioabsorbable material (B), and wherein at least the non-bioabsorbable material is retrieved after implantation of the device.

28. The method according to claim 27, wherein the retrieval is performed without causing bleeding, inflammation and/or rupture of a blood vessel.

29. An implantation method comprising implanting a cell- or tissue-containing device into a site in which the device according to any one of claims 1 to 22 has been implanted.

30. A method for preventing and/or treating a disease or a symptom, comprising implanting a cell- or tissue-containing device into a site in which the device according to any one of claims 1 to 22 has been implanted.

31. The method according to claim 29 or 30, further comprising implanting the device according to any one of claims 1 to 22.

32. The device or method according to any one of claims 1 to 31, wherein the implantation site is a subcutaneous site.
